# FASCICULE DE BREVET EUROPEEN

(11) **EP 0 389 347 B1**
(45) Date de publication et mention de la délivrance du brevet: **08.10.2003**
(21) Numéro de dépôt: 90400734.1
(22) Date de dépôt: 19.03.1990
(51) Int. Cl.: C12N 5/00, A61K 39/108, A61K 39/112, C12N 15/31

(54) **Vaccins contre les bactéries septicémiques**
Impfstoffe gegen Bakterien, die zur Blutvergiftung führen
Vaccines against septicemic bacteria

(30) Priorité: 20.03.1989 FR 8903626
(43) Date de publication de la demande: 26.09.1990
(73) Titulaire: MERIAL, 69002 Lyon (FR)
(72) Inventeur: Audonnet, Jean-Christophe, F-26200 Montélimar (FR); Bruneau, Patrick, F-69110 Sainte Foy les Lyon (FR)
(74) Mandataire: Bernasconi, Jean

(56) Documents cités:
- CHEMICAL ABSTRACTS, vol. 97, 1982, page 291, résumé no. 35849p, Columbus, Ohio, US; A. BINDEREIF et al.: "The cloacin receptor of CoIV-bearing Escherichia coli is part of the iron(3+)-serobactin transport system", & J. BACTERIOL. 1982, 150(3), 1472-5
- IDEM
- BIOLOGICAL ABSTRACTS, vol. 84, no. 3, 1987, résumé no. 25347, Biological Abstracts, Inc., Philadelphia, PA, US; C.A. BOLIN et al.: "Passive immunization with antibodies against iron-regulated outer membrane proteins protects turkeys from Escherichia coli septicemiq", & INFECT IMMUN 55(5): 1239-1242. 1987
- CHEMICAL ABSTRACTS, vol. 103, 1985, page 361, résumé no. 68057z, Columbus, Ohio, US; E. GRIFFITHS et al.: "Synthesis of acrobactin and a 76,000-dalton iron-regulated outer membrane protein by Escherichia coli K-12 Shigella flexneri hybrids and by enteroinvasive strains of Escherichia coli", & INFECT. IMMUN. 1985, 49(1), 67-71
- CHEMICAL ABSTRACTS, vol. 107, 1987, page 161, résumé no. 148286w, Columbus, Ohio, US; C.L. MAROLDA et al.: "Flanking and internal regions of chromosomal genes mediating aerobactin iron uptake systems in enteroinvasive Escherichia coli and Shigella flexneri", & J. GEN. MICROBIOL. 1987, 133(8), 2269-78
- CHEMICAL ABSTRACTS, vol. 98, 1983, page 176, résumé no. 120604v, Columbus, Ohio, US; A. BINDEREIF et al.: "Cloning of the aerobactin-mediated iron assimilation system of plasmid CoIV", & J. BACTERIOL. 1983, 153(2), 1111-13
- CHEMICAL ABSTRACTS, vol. 105, 1986, page 154, résumé no. 92124f, Columbus, Ohio, US; V. DE LORENZO et al.: "Aerobactin biosynthesis and transport genes of plasmide CoIV-K30 in Escherichia coli K-12", && J. BACTERIOL. 1986, 165(2), 570-8
- CHEMICAL ABSTRACTS, vol. 95, 1981, page 306, résumé no. 93711g, Columbus, Ohio, US; P.J. WARNER et al.: "CoIV plasmid-specified aerobactin synthesis by invasive strains of Escherichia coli", & INFECT. IMMUN. 1981, 33(2), 540-5
- BIOLOGICAL ABSTRACTS, vol. 81, no. 9, 1986, résumé no. 81571, Biological Abstracts, Inc., Philadelphia, PA, US; M.D. MAGAZIN et al.: "The outer membrane receptor protein for ferric pseudobactin, a sideropbore from a plant growth-promoting Pseudomonas strain", & J. BIOL. CHEM. 261(2), 795-799
- BIOLOGICAL ABSTRACTS, vol. 77, no. 10, 1984, résumé no. 75003, Biological Abstracts, Inc., Philadelphia, PA, US; L. FECKER et al.: "Cloning and expression of the fhu genes involved in iron(III)-hydroxamate uptake y Escherichia coli", & J. BACTERIOL 156(3): 1301-1314
- LIN J. ET AL J. DAIRY SCI. vol. 81, no. 8, 1998, pages 2151 - 2158

## Description

La présente invention a trait à des bactéries appartenant aux genres incluant des bactéries pathogènes et exprimant en grandes quantités des protéines antigéniques de la membrane externe régulées par le fer.

Elle concerne également les procédés pour la production de ces bactéries.

Elle concerne encore des vaccins anti-bactéries septicémiques contenant à titre de principe actif des bactéries exprimant en grandes quantités des protéines antigéniques de la membrane externe régulées par le fer, ou des fragments de ces bactéries ou des antigènes exprimés par ces bactéries.

Elle concerne également d'autres vecteurs bactériens, viraux, ou autres, permettant d'exprimer ces protéines antigéniques, ainsi que des vaccins contenant ces protéines à titre de principe actif.

Elle concerne également des vaccins vivants recombinants, notamment bactériens ou viraux, exprimant ces protéines antigéniques dans l'organisme vacciné.

On sait qu'à l'exception de certains lactobacilles, le fer est un nutriment nécessaire à toutes les formes vivantes, y compris les bactéries. Celles-ci ont besoin de fer pour pouvoir se multiplier dans la cellule hôte. L'aptitude de la bactérie à se multiplier in vivo constitue un facteur essentiel de la virulence.

Bien que le fer soit présent en grandes quantités dans l'organisme humain, la bactérie ne dispose pour se multiplier que d'une quantité très restreinte de fer libre.

La très grande majorité du fer d'un hôte animal est, en effet, intracellulaire (sous forme de ferritine, d'hémosidérine ou d'hème) et donc difficilement accessible. La faible quantité de fer présente dans les liquides corporels n'existe que sous forme de complexes extrêmement stables, constitués principalement avec deux glycoprotéines chélatrices du fer : la transferrine dans le plasma et la lactoferrine dans les sécrétions. L'existence de ces glycoprotéines qui lient le fer avec force, mais de façon réversible, est nécessaire pour permettre son utilisation par les cellules, tout en empêchant sa précipitation sous forme d'hydroxyde ferrique.

Le plasma contient des complexes de fer sous forme d'haptoglobine-hème, de céruloplasmine, de ferritine, de lactoferrine et de transferrine.

La majeure partie du fer est véhiculée par les transferrines. On distingue trois classes majeures de transferrine : la transferrine sérique, la lactoferrine et l'ovotransferrine.

La transferrine fixe environ 95 % du fer plasmatique et son taux de saturation est seulement d'environ 35 % chez un individu sain.

La lactoferrine a un très faible taux de saturation en fer et elle conserve ses propriétés chélatrices dans une large gamme de pH ; sa présence dans toutes les sécrétions de l'organisme, c'est-à-dire au niveau des sites potentiels d'invasion microbienne, impose une plus grande restriction en fer à ces endroits qu'ailleurs dans l'organisme.

La complexation du fer aux glycoprotéines a pour conséquence de ne laisser subsister qu'une très faible concentration de fer ferrique libre (10⁻¹⁹M), qui est tout à fait insuffisante pour permettre la croissance normale des bactéries.

Afin d'acquérir le fer dont elles ont besoin pour se multiplier chez l'hôte, les bactéries disposent d'un certain nombre de moyens.

Certains microorganismes semblent pouvoir obtenir leur fer par un mécanisme impliquant une interaction directe entre la surface cellulaire bactérienne et les protéines liant le fer chez l'hôte. Ce mode d'acquisition direct ne concerne toutefois qu'un nombre très limité d'espèces. La plupart des bactéries, qu'elles soient pathogènes ou non, combattent l'indisponibilité du fer, chez l'hôte ou dans certains environnements aérobies, en produisant des composés chélateurs du fer appelés sidérophores.

Les sidérophores sont constitués par des molécules de faible poids moléculaire qui forment des complexes spécifiques et de très grande affinité avec l'ion ferrique. Leur biosynthèse est régulée par le fer et leur fonction est d'approvisionner la cellule bactérienne en fer.

Ces sidérophores possèdent une affinité extrêmement élevée pour l'ion ferrique (leur constante d'association est d'environ 10³⁰ M⁻¹) qui leur permet de déplacer le fer associé aux protéines de l'hôte ou de solubiliser le fer ferrique précipité sous forme d'hydroxyde.

La plupart des sidérophores jusqu'ici identifiés appartiennent à deux classes chimiques : les phénolates-catécholates (dérivés de l'acide 2,3 dihydroxybenzoïque) et les hydroxamates (dérivés de l'acide hydroxamique).

Le plus connu des sidérophores de la classe des phénolates est l'entérobactine excrétée par les bactéries des genres Escherichia, Klebsiella, Salmonella et Shigella. L'entérobactine est constituée d'un trimère cyclique de la 2,3 dihydroxy-N-benzoyl-L-sérine et est le composé chimique qui possède la plus haute affinité connue pour l'ion ferrique (Ka = 10⁵²M⁻¹).

Plusieurs espèces entériques synthétisent un autre sidérophore hydroxamate, (l'aérobactine. Ce sidérophore est, en particulier, produit par des souches septicémiques ou invasives d'Escherichia coli comportant un plasmide de *type* Col V, de Salmonella typhymurium et de Shigella.

Cette biosynthèse des sidérophores par les bactéries est associée à la production de protéines au niveau de la membrane externe dont certaines servent de récepteurs aux sidérophores ainsi que de mécanismes qui permettent le transport et le relargage du fer à l'intérieur de la bactérie. Ces protéines formées dans la membrane externe, souvent désignées par le terme "IROMP" pour Iron Regulated Outer MembraneProtein (protéine de membrane externe régulée par le fer), ont pour caractéristique commune d'avoir une taille comprise entre 70 kDa et 90 kDa et d'ère synthétisées aussi bien in vitro en milieu restreint en fer qu'in vivo au cours de l'infection.

Les protéines de la membrane externe, récepteurs de sidérophores, constituent donc le deuxième élément des systèmes dits de haute affinitié pour l'acquisition bactérienne du fer (le premier élément étant constitué par les sidérophores).

A côté de ces systèmes de haute affinité, de nombreuses bactéries disposent de systèmes de transport de basse affinité qui leur permettent d'utiliser les formes polymérisées d'hydroxyde ferrique.

Les mécanismes d'assimilation du fer ont été particulièrement étudiés chez Escherichia coli qui est le microorganisme le mieux connu sur le plan génétique.

Le système endogène de haute affinité de transport du fer chez E. coli utilise le sidérophore, entérobactine. L'entérobactine est synthétisée et excrétée dans le milieu lorsque E. coli est placée dans un environnement restreint en fer. Les complexes d'entérobactine ferrique sont alors captés au niveau de la membrane externe (protéine Fep A de 81 kDa) et transportés vers le cytoplasme. Une fois internalisé, le fer est libéré par hydrolyse de l'entérobactine ferrique, puis réduit en fer ferreux.

Le système entérobactine de E. coli comprend au moins treize gènes. Sept gènes (ent) sont impliqués dans la biosynthèse du sidérophore et cinq gènes (fep) codent pour des protéines de transport.

En plus du système entérobactine, les souches septicémiques d'E. coli excrètent et transportent un sidérophore hydroxamate, l'aérobactine.

Il a été découvert en 1979 par P.H. WILLIAMS (37) que certains plasmides de type Col V portaient les gènes du sidérophore aérobactine et de son récepteur situé dans la membrane externe et appelé protéine Iut A (protéine de 74 kDa).

Bien que l'aérobactine ait une constante d'association avec l'ion ferrique inférieure à celle de l'entérobactine, elle est néanmoins douée de propriétés structurales qui augmentent sa capacité à récupérer le fer lié à la transferrine ou à la lactoferrine.

Depuis sa mise en évidence en 1979 par P.H. WILLIAMS, de très nombreuses études ont montré que le système de transport du fer par l'aérobactine jouait un rôle déterminant dans la virulence des souches pathogènes d'E. coli et de nombreuses autres bactéries (GRIFFITHS et al (13)).

La présence du sidérophore aérobactine favorise fortement la virulence des souches pathogènes.

Si l'aérobactine est un chélateur moins puissant que l'entérobactine, il est par contre actif dans des conditions d'environnement beaucoup plus variées (l'entérobactine est très sensible à l'oxydation et aux variations de pH). L'aérobactine assure donc à la bactérie un degré d'adaptation plus grand.

Par ailleurs, l'aérobactine stimule mieux la croissance bactérienne et elle semble être excrétée beaucoup plus rapidement que l'entérobactine, probablement par suite d'une induction génétique préférentielle, lorsque E. coli est cultivée en présence d'un chélateur.

La bactérie acquiert, avec l'opéron aérobactine, un système de transport du fer extrêmement performant avec un nombre minimum de gènes supplémentaires, quatre gènes seulement pour la synthèse de l'aérobactine qui est un petit sidérophore simple, et un gène codant pour le récepteur de la membrane externe. Les autres gènes nécessaires au -transport des hydroxamates sont, en effet, constitutivement présents chez toutes les Escherichia coli.

L'expression de tous les gènes codant pour les protéines de membrane, récepteurs de sidérophores, et les sidérophores correspondants, est régulée par une seule protéine, Fur, qui agit comme un répresseur lorsque le fer est disponible en quantité suffisante. Cette régulation centrale est surimposée à une régulation individuelle qui module l'expression de chaque système selon les conditions d'environnement.

Certains auteurs ont cultivé des bactéries, en vue d'augmenter l'expression d'IROMPs dans des milieux carencés en fer à l'aide de chélateurs chimiques, tels que le α, α' dipyridyl (A. BINDEREIF et al. : The cloacin receptor of Col V-bearing Escherichia coli is part of the Fe 3+ aerobactin system, J. Bacteriol., 1982, 150, 1472-1475 ; C. MAROLDA et al. : Flanking and internai regions of chromosomal genes mediating aerobactin iron uptake system in enteroinvaisve Escherichia coli and Shigella flexneri, J. General Microbiology, 1987, 133, 2269-2278 ; A. BINDEREIF et al. : Cloning of the aerobactin-mediated iron assimilation system of plasmid col V, J. Bateriol., 1983, 153, 1111-1113; De LORENZO et al. : Aerobactin biosynthesis and transport genes of plasmid col V - K 30 in Escherichia coli K 12, J. Bacteriol., 1986, 165, 570-578 ; P. WARNER et al. : col V-plasmid-specified aerobactin synthesis by invasive strains of Escherichia coli, Infection and Immunity, 1981, 33, 540-545. E. GRIFFITHS et al. ont montré dans : Synthesis of aerobactin and a 76000 Daltons iron-regulated outer membrane protein by Escherichia coli K-12 - Shigella flexneri hybrids and by enteroinvasive strains of Escherichia coli, Infection and Immunity, 1985, 49, 67-71, que des souches entéroinvasises de E. coli produisent de l'aérobactine et une protéine de membrane externe de 76 K en culture réduite en fer en présence d'ovotransferrine.

Les connaissances récemment acquises sur les systèmes d'acquisition du fer par les bactéries ont permis l'exploration de nouvelles voies de lutte contre les bactéries pathogènes.

Il a été proposé de synthétiser des analogues de sidérophores qui soient toxiques pour la bactérie et puissent leurrer les systèmes de transport du fer pour entrer dans la cellule bactérienne. Mais ces chélateurs synthétiques ont une affinité pour le fer (III) moins élevée que les sidérophores naturels, et sont incapables de déplacer le fer des transferrines.

ROGERS a suggéré la formation de complexes entre l'aérobactine et des ions métalliques trivalents pour les utiliser comme antimétabolites vis-à-vis du complexe naturel entérobactine-Fe³⁺. Seuls les complexes formés avec le scandium (Sc³⁺) et l'indium (In ³⁺ ) présentent une certaine activité antibactérienne (ROGERS et al (26) ; ROGERS (27)).

Il a été également proposé d'adsorber les sidérophores de type phénolate qui sont des molécules aromatiques sur certaines protéines sériques, qui jouent alors le rôle de molécule porteuse, permettant l'induction d'anticorps spécifiques dirigés contre le sidérophore.

C'est ainsi que BYERS (5) décrit un vaccin contre le sidérophore phénolate produit par Aeromonas hydrophila (bactérie responsable de septicémie chez l'homme et chez le poisson), qui a été expérimenté chez les poissons. Le sidérophore a été couplé de façon covalente à l'albumine humaine ou à l'albumine bovine. Des poissons immunisés avec ces préparations produisent des anticorps qui réagissent contre le sidérophore. Il n'est, toutefois, pas précisé si les anticorps formés sont aptes à neutraliser le sidérophore.

On a pensé, également, empêcher le captage des sidérophores par les bactéries par des anticorps qui seraient spécifiquement dirigés contre les récepteurs de sidérophores.

BOLIN et al (4) rapportent les résultats d'une étude qui fait état d'une certaine immunisation passive avec des anticorps dirigés contre les protéines de la membrane externe régulées par le fer, protégeant des dindons d'une septicémie par Escherichia coli.

Toutes les tentatives entreprises en vue de la mise au point d'un vaccin efficace ont, toutefois, jusqu'ici échoué, devant les difficultés à pouvoir faire exprimer par une bactérie les protéines de membrane régulées par le fer à un niveau suffisant dans la culture bactérienne.

La présente invention permet de surmonter cette difficulté, en proposant l'utilisation dans un vaccin, à titre de principe actif, de bactéries exprimant en grandes quantités des protéines de membrane régulées par le fer et plus particulièrement, les récepteurs de sidérophores à un niveau suffisant pour induire la formation d'anticorps qui empêchent la reconnaissance spécifique des sidérophores par leurs récepteurs.

Un des buts de l'invention est de fournir des bactéries exprimant des protéines de la membrane externe régulées par le fer (IROMPs), utilisables comme antigènes protecteurs.

Un autre but de l'invention est de proposer la synthèse des protéines de la membrane externe régulées par le fer de bactéries septicémiques par recombinaison génétique.

Un autre but de l'invention est de fournir en grande quantité les IROMPs et notamment les protéines Iut A et Fep A, récepteurs des sidérophores, aérobactine et entérobactine chez Escherichia coli et d'autres familles, par synthèse de ces protéines par recombinaison génétique.

Un autre but encore de l'invention est de fournir des vaccins qui contiennent, à titre de principe actif, des bactéries ou des fragments de ces bactéries qui présentent dans leur membrane externe de grandes quantités d'IROMPS et notamment des protéines Iut A et Fep A, obtenues par recombinaison génétique ou par d'autres procédés.

Un autre but encore de l'invention est de fournir des vaccins qui contiennent, à titre de principe actif, des IROMPs, par exemple des protéines Iut A et/ou Fep A ou des préparations d'antigènes incorporant ces protéines.

La présente invention utilise donc des bactéries exprimant des protéines de la membrane externe régulées par le fer et dont certaines sont des récepteurs de sidérophores, utilisables dans une préparation vaccinale.

Les bactéries conformes à l'invention sont caractérisées en ce qu'elles expriment des quantités accrues de ces protéines de la membrane externe, et plus particulièrement des récepteurs de transferrines et notamment des récepteurs de sidérophores, pour induire, lorsque ces protéines sont utilisées dans un vaccin, la formation d'anticorps empêchant la fonction de reconnaissance spécifique par les récepteurs, et arrêtant par là même l'alimentation en fer de la bactérie pathogène.

Les bactéries utilisées sont, de préférence, des entérobactéries et on préfère qu'elles excrètent des sidérophores entérobactine et/ou aérobactine.

Les bactéries sont choisies, de préférence, dans le groupe constitué par Escherichia coli, Klebsiella, Salmonella thyphimurium, Shigella.

Les bactéries excrètent, de préférence, ensemble les sidérophores aérobactine et entérobactine.

Conformément à l'invention, et selon un premier mode de réalisation, les bactéries sont obtenues par culture de souches existantes dans la nature ou disponibles dans les laboratoires ou collections dans un milieu minimum dans lequel la disponibilité du fer est réduite à un niveau permettant une expression accrue satisfaisante des protéines de membrane. La culture est effectuée, de préférence, en présence d'une protéine forte chélatrice de fer (III) telle que les lactoferrines, chélateurs qui présentent l'avantage d'établir une restriction en fer ayant les mêmes caractéristiques que celle qui existe in vivo.

L'invention a également pour objet un procédé de production de telles bactéries destinées à être utilisées pour la préparation de vaccins, caractérisé en ce que l'on cultive lesdites bactéries dans un milieu de culture contenant une protéine chélatrice du fer (III) telle que les transferrines, et notamment lactoferrines.

On peut utiliser, comme milieu minimum, celui qui a été décrit par exemple par SIMON et TESSMAN (30).

Ce mode de réalisation reste, toutefois, d'une mise en oeuvre délicate, car il faut, avec les chélateurs du fer généralement utilisés, réduire suffisamment la teneur en fer dans le milieu de culture, pour obtenir une expression suffisante des protéines de la membrane externe. Il reste souvent de faibles quantités de fer qui empêchent l'expression des protéines de membrane (fer provenant, par exemple, du fermenteur ou des tuyauteries qui sont, en général, en acier inoxydable). On est, alors, obligé d'avoir recours à des procédés relativement compliqués, pour abaisser la teneur en fer à un niveau qui permette l'expression des protéines de la membrane externe des bactéries, et *dont* la mise en oeuvre est coûteuse.

Selon un second mode de réalisation de l'invention, qui est aussi le mode de réalisation préféré, les bactéries exprimant, en grandes quantités, les protéines de la *mem*brane externe, récepteurs de sidérophores, sont tranformées par des plasmides recombinants.

La synthèse des protéines de la membrane externe, récepteurs de sidérophores ou de transferrine, par recombinaison génétique, présente, en effet, de nombreux avantages :
- elle permet d'avoir une expression importante de ces protéines, indépendamment de la concentration en fer du milieu de culture,
- elle permet d'étudier les réactions immunitaires directement dirigées contre ces protéines, en excluant tout autre constituant de la souche d'origine,
- elle est la solution la plus économique pour exprimer des protéines de membrane dans un environnement où le fer est toujours présent (fermenteurs, tuyauteries et équipements divers en acier inoxydable).

Si le demandeur s'est plus particulièrement intéressé à la synthèse des protéines Iut A et Fep A, récepteurs des sidérophores aérobactine et entérobactine chez E. coli, on comprendra que les techniques de recombinaison génétique décrites ci-après, s'appliqueront par analogie à la synthèse des protéines de membrane (IROMPs), récepteurs des sidérophores, (aérobactine et entérobactine) ou de transferrines de bactéries pathogènes autres que E. coli.

L'invention concerne donc aussi la préparation des protéines Iut A et/ou Fep A d'E. coli par recombinaison génétique.

La protéine Iut A peut être synthétisée par un procédé qui consiste, notamment :
- à isoler le plasmide ou le chromosome de souches d'E. coli de Salmonelles, Shigelles ou Klebsielles pathogènes qui porte l'opéron aérobactine,
- à séparer du plasmide ou chromosome un fragment qui contient le gène iut A,
- à liguer ledit fragment avec un vecteur de clonage,
- à insérer les clones ayant intégré le gène iut A dans un vecteur d'expression (par exemple le plasmide GTI 001),
- et à exprimer la protéine IutA par culture des clones.

La protéine Fep A est obtenue :
- en isolant d'un plasmide (par exemple le plasmide pMS 101 construit par LAIRD et YOUNG (19)) ou d'un chromosome bactérien, E. coli, Salmonelles ou Klebsielles un fragment portant le gène fep A,
- en clonant ledit fragment dans un vecteur de clonage,
- en insérant le gène fep A dans un vecteur d'expression, de préférence, dans le vecteur utilisé pour l'expression de la protéine Iut A (plasmide GTI 001),
- et en faisant exprimer la protéine Fep A par culture des clones.

Les vecteurs d'expression des protéines Iut A et/ou Fep A peuvent être des bactéries et on préférera utiliser E. coli dont les systèmes d'expression sont les mieux connus. On peut cependant également utiliser d'autres vecteurs, notamment viraux ou constitués de levures, dont les constructions peuvent être mises en oeuvre par l'homme de l'art.

Les clones bactériens exprimant les protéines Iut A et/ou Fep A peuvent être multipliés, dans un milieu approprié, à une température suffisamment basse pour empêcher ou limiter l'expression, en général inférieure à 32°C. L'expression est ensuite induite par élévation de La température, par exemple à 42°C pendant environ 4 heures, pour induire l'expression des gènes iut A et fep A.

On obtient ainsi des bactéries qui intégrent les protéines Iut A et Fep A ainsi que leurs précurseurs proIut A et proFep A, se présentant sous la forme d'inclusions cytoplasmiques de grande taille.

Ces bactéries utilisées, dans un vaccin, à titre de principe actif, induisent la formation d'anticorps dirigés contre les protéines Iut A et Fep A qui empêchent la reconnaissance par ces protéines de leurs sidérophores respectifs aérobactine et entérobactine, réduisant fortement par là même, l'alimentation en fer de la bactérie et bloquant sa multiplication.

L'invention concerne donc également des vaccins qui contiennent, à titre de principe actif, des bactéries recombinantes exprimant des protéines de la membrane externe régulées par le fer.

Elle a plus particulièrement pour objet des vaccins qui contiennent, à titre de principe actif: des bactéries recombinantes ou des fragments de ces bactéries, notamment des fragments de membranes qui intègrent des protéines Iut A et/ou Fep A et/ou leurs précurseurs proIut A et/ou proFep A; ou encore les protéines Iut A et/ou Fep A et/ou leurs précurseurs, par exemple extraits du cytoplasme ou de la membrane externe des bactéries recombinantes.

Dans un autre mode de mise en oeuvre, l'invention a pour objet des vaccins qui contiennent, à titre de principe actif: des bactéries homologues des bactéries septicémiques, ou des fragments de ces bactéries, cultivées dans un milieu à restriction de fer, et qui intègrent, en quantités accrues, des protéines Iut A et/ou Fep A et/ou encore leur précurseurs ; les protéines Iut A et/ou Fep A (et/ou leurs percurseurs) convenablement extraites.

De préférence, ces derniers vaccins sont préparés à base de bactéries cultivées dans un milieu contenant un chélateur de fer (III) fort de type protéique et notamment transferrine, lactoferrine ou ovotransferrine.

L'invention sera bien comprise à la lecture de la description qui suit, se référant aux figures ci-annexées sur lesquelles :
la figure 1 représente le profil protéique obtenu par électrophorèse d'un clone exprimant la protéine Iut A,
la figure 2 représente le profil protéique obtenu par électrophorèse d'un clone exprimant la protéine Fep A.

Les abréviations utilisées dans la suite du texte ont les significations qui suivent :
- Amp^{r}: ampicilline-résistant
- Clo^{s}: cloacine-sensible
- dATP: désoxy-adénosine triphosphate
- EDTA: acide éthylène-diamine-tetraacétique
- Ent: entérobactine
- E.O.P.S.: exempt d'organismes pathogènes spécifiques
- IPTG: isopropyl-β-thiogalactopyranoside
- kpb: kilopaire de bases
- LB: luria broth
- OMP: protéine extra-membranaire (outer membrane protein)
- PAGE: électrophorèse en gel de polyacrylamide
- pb: paire de bases
- PBS: tampon phosphate (phosphate buffered saline)
- SDS: sodium dodecyl sulfate
- ST: Simon et Tessman
- TEMED: M,N,M',N'-tétraméthylène diamine
- Tris: tris-hydroxy-aminométhyl-méthane
- tet^{r}: tétracycline-résistant

### MATERIELS ET METHODES

### I. MATERIELS

### 1. Souches

On a rassemblé dans le tableau I les souches utilisées. Les souches pathogènes mises en oeuvre sont des souches de septicémies chez des veaux ou des poulets et proviennent de la souchothèque RHONE-MERIEUX.

Les souches hôtes utilisées pour le clonage, le séquençage et l'expression dérivent toutes de Escherichia coli K 12.

On peut, sans difficultés, remplacer ces souches, par d'autres souches septicémiques sauvages ou des souches de laboratoire

### 2. Plasmides

L'origine et les caractéristiques des plasmides utilisés pour le clonage et l'expression sont présentées dans le tableau II.

### 3. Milieux

Milieu minimum de SIMON et TESSMAN (30).

Sa composition est la suivante :

| | | |
|---|---|---|
| NaCl | 5,8 g | |
| KCl | 3,7 g | |
| CaCl₂, 2H₂O | 0.15 g | |
| MgCl₂, 6H₂O | 0,10 g | |
| NH₄Cl | 1,10 g | |
| Na₂SO₄ | 0,142 g | |
| KH₂PO₄ | 0,272 g | |
| Tris | 11,20 g | |
| H₂O qsp | 1000 ml | pH 7,4 |

L'unique source de carbone est constituée par du succinate de sodium ajouté à la concentration finale de 10 g/l.

Pour établir une restriction en fer dans ce milieu,on a ajouté de l'ovotransferrine (SIGMA) à la concentration finale de 250 µg/ml (Des concentrations supérieures à 500 µg/ml sont possibles).

Le milieu témoin riche en fer est obtenu par addition de FeCl₃, 6H₂O (MERCK) à la concentration finale de 40µM.

Milieu minimum M9 MANIATIS (29)

| | | |
|---|---|---|
| Na₂HPO₄ | 6,0 g | |
| KH₂PO₄ | 3,0 g | |
| NaCl | 0,5 g | |
| MH₄Cl | 1,0 g | |
| H₂O qsp | 1000 ml | pH 7,4 |

on ajoute à ce milieu de base :

| | |
|---|---|
| Mg SO₄ 1M | 2 ml/1000 ml |
| glucose 20 % | 10 ml/1000 ml |
| CaCl₂ 1M | 0,1 ml/1000 ml |

- Milieu riche LB (MANIATIS et al., (23))

| | | |
|---|---|---|
| Bactotryptone | 10 g | |
| Extrait de levure | 5 g | |
| MaCl | 5 g | |
| H₂O qsp | 1000 ml | pH 7,4 |

- Milieu riche BTS (BIO MERIEUX)

| | |
|---|---|
| Biotryptase | 17 g |
| Biosoyase | 3 g |
| NaCl | 5 g |
| K₂HPO₄ | 2,5 g |
| glucose | 2,5 g |
| H₂O qsp | 1000 ml |

- Milieu riche BHI (Coeur-cervelle BIO MERIEUX)

| | | |
|---|---|---|
| Infusion de cervelle de veau | 200 g | |
| Infusion de coeur de boeuf | 250 g | |
| Bio-gelytone | 10 g | |
| NaCl | 5 g | |
| Na₂HPO₄ | 2,5 g | |
| glucose | 2,0 g | |
| H₂O qsp | 1000 ml | pH 7,4 |

- Milieu riche "M9 SP" pour l'expression milieu :

| | |
|---|---|
| SP (Tryptone 3,2 % ; Extrait de levure 2 %) | 100 ml |
| M9 (6,6 X concentré) filtré sur filtre de 0,22 µm (Millipore) | 15 ml |
| Mg SO₄ 100 mM | 1,5 ml |
| FeCl₃ 0,1 mM | 1,5 ml |
| Vitamine B1 (solution à 5 %) | 1,5 ml |

Les milieux solides ont la même composition que les milieux liquides correspondants et contiennent 12 g d'agar par litre de milieu.

Les antibiotiques sont utilisés dans les milieux solides et liquides aux concentrations finales suivantes :

| | |
|---|---|
| Ampicilline | 25 ug/ml |
| Tétracycline | 12,5 ug/ml |

L'isopropyl-β -D-thiogalactopyranoside (IPTG) est ajouté, selon les cas, à une concentration finale allant de 0,05 mM à 0,4 mM.

La stérilisation des milieux liquides et solides est effectuée par autoclavage à 120°C pendant 20 minutes.

Les solutions d'antibiotiques, de vitamine B1, de succinate de sodium, de M9 6,6 X, de MgSO₄, de FeCl₃, d'IPTG et d'ovotransferrine sont réalisées sous forme de solutions stocks concentrées, et stérilisées par filtration sur filtre de porosité 0,22 µm (Millipore).

Après stérilisation, les milieux de culture sont conservés à température ambiante.

Les solutions stocks d'antibiotiques, d'IPTG et d'ovotransferrine sont conservées à -20°C.

Les autres solutions sont gardées à + 4°C.

### II METHODES

### 1. Cultures bactériennes

A l'exception des clones abritant les constructions réalisées dans le vecteur d'expression pGTI 001, qui sont cultivés à + 30°C, toutes les cultures sont effectuées à + 37°C, sous agitation, pendant 18 heures.

Lorsque cela est nécessaire, la croissance bactérienne est estimée par la mesure de la turbidité de la suspension à 600 nm, à l'aide d'un spectrophotomètre BECKMAN DU 40.

Les cultures sont habituellement réalisées sous un volume de 2 ml après ensemencement avec une colonie. Les cultures sous un plus grand volume (20 ml à 1000 ml) sont réalisées par ensemencement au 1/100ème avec une préculture en phase stationnaire.

### 2. Sensibilité vis-à-vis des bactérocines

Les productions de cloacine DF 13 et de colicine B sont réalisées respectivement avec les souches Enterobacter cloacae DF 13 et Escherichia coli 1300 selon la méthode décrite par DE GRAAF (DE GRAAF et al (8) et (9)).

Ces souches sont cultivées à + 37°C, en milieu BHI, jusqu'à ce que la densité optique atteigne 0,5 (1 cm, 600 nm). On ajoute alors au milieu de culture de la mitomycine C, de façon à avoir une concentration finale de 1 µg/ml, ce qui permet l'induction de la synthèse *des* bactériocines. La culture est continuée pendant 6 heures, à + 37°C, jusqu'à la phase de lyse. Les corps bactériens sont centrifugés (8000 g, 30 mn, + 4°C) et le surnageant est récolté. On additionne alors lentement, à + 4°C, du sulfate d'ammonium jusqu'à ce que la concentration atteigne 365 g/l.

Le surnageant est repris en tampon phosphate 0,05 M pH 7,0 et dialysé contre plusieurs bains successifs de ce tampon. Le dialysat est filtré sur 0,22 µm (Millipore) et conservé à - 20°C.

Environ 10⁹ bactéries du clone étudié sont étalées sur une gélose LB contenant l'antibiotique de sélection approprié. Lorsque le liquide du dépôt a été complètement absorbé, on dépose au centre de la boîte de Petri 75 µl de la solution de bactériocine. Lorsque cette goutte a elle-même séché, la boîte de Petri est placée à l'étuve (+ 30°C ou + 37°C selon les cas) pendant 18 heures. Les clones qui présentent une inhibition de croissance au niveau du dépôt ont acquis la sensibilité à la bactériocine. Les clones résistants à l'action toxique des bactériocines présentent au contraire un tapis bactérien uniforme.

### 3. Préparation d'antisérums dirigés contre les protéines de la membrane externe régulées par le fer

Le protocole utilisé reproduit celui qui a été décrit par BOLIN et JENSEN (4).

Les protéines de la membrane externe régulées par le fer sont séparées par électrophorèse préparative sur gel de polyacrylamide, en présence de dodécyl sulfate de sodium.

Après coloration des gels, la bande contenant l'IROMP devant servir à l'immunisation est découpée, puis broyée en présence d'eau distillée, par passages successifs à travers des aiguilles de diamètre de plus en plus faible.

Ce broyat est injecté à des lapins E.O.P.S. de souche White New Zealand, provenant de l'élevage de la société Rhône-Mérieux, selon le protocole présenté dans le tableau III.

Pour éliminer les anticorps dirigés contre les autres protéines de la membrane externe, les lipopolysaccharides et les autres antigènes de Escherichia coli, les sérums récoltés chez les lapins immunisés sont adsorbés avec une souche d'Escherichia coli n'exprimant pas d'IROMPs.

A chaque ml de sérum, on ajoute environ 10¹⁰ corps bactériens inactivés par la chaleur pendant 30 mn à 100°C, et on incube le mélange à + 37°C pendant 1 heure.

Après centrifugation, le sérum surnageant est récupéré et filtré sur filtre de porosité 0,22 µm (Millipore).

Il est conservé à - 20°C.

### 5. Extraction des protéines de la membrane externe.

La technique utilisée pour extraire les protéines de la membrane externe dérive des techniques décrites par VAN TIEL-MENKVELD et al. (36) et par FISS et al. (12)

Après centrifugation, le culot bactérien est remis en suspension dans du tampon (Tris/HCl 0,2M pH 8,0 de façon à obtenir une densité optique d'environ 10. Les cellules bactériennes sont alors éclatées par sonication en faisant agir les ultrasons 3 fois 3 minutes en maintenant la suspension bactérienne sur un bain glace-éthanol.

Les débris cellulaires et les bactéries intactes sont éliminées s par centrifugation à 5000 g pendant 20 mn, à + 4°C.

Les membranes bactériennes en suspension dans le surnageant sont récupérées par ultracentrifugation à 110 000 g pendant 60 minutes à + 4°C.

Le culot membranaire est repris par 5 ml du tampon d'extraction décrit par SCHNAITMAN (29) : Triton X-100 2%; MgCl₂ 10 mM ; Tris/HCl 50 mM pH 8,0.

On incube 30 minutes, à température ambiante, en agitant toutes les 5 minutes. Au cours de l'incubation, les protéines de la membrane cytoplasmique sont préférentiellement solubilisées par le Triton X-100. Les membranes externes sont récupérées par une nouvelle ultracentrifugation (110 000 g, 60 mn, + 4°C). Le culot obtenu est lavé 3 fois en eau distillée, resuspendu finalement dans 1 ml d'eau distillée et congelé à - 20°C pour conservation.

### 6. Dosage des protéines.

La concentration protéique des extraits membranaires est mesurée par une méthode colorimétrique dérivée de celle publiée par LOWRY et al. (20).

A 0,5 ml de la solution protéique à doser, sont ajoutés 2,5 ml de la solution suivante :

| | |
|---|---|
| Solution de CuSO₄ à 1 % | 1 ml |
| Solution de tartrate de sodium à 2 % | 1 ml |
| Solution de carbonate de sodium à 2% en NaOH 0,1N qsp | |

Après incubation de 10 minutes à température ambiante, on ajoute 0,25 ml de réactif de Folin (Merck) à 50 %.

On incube 30 minutes, à température ambiante, et on mesure la densité optique de la coloration bleue, qui s'est développée, à 779 nm.

On détermine la concentration protéique des échantillons à l'aide d'une gamme étalon préparée avec de l'albumine sérique de boeuf.

La densité optique est proportionnelle à la concentration protéique dans l'intervalle de 5 à 200 µm/ml.

### 7. Technique d'analyse de la composition protéique des membranes externes : électrophorèse sur gel de polyacrylamide en conditions dénaturantes.

Les gels de polyacrylamide sont préparés selon les caractéristiques décrites par LUGTENBERG et al. (21).

Le gel d'alignement a la composition suivante :
acrylamide-bisacrylamide (30/0,8 p/p) 5%; Tris/HCl 130 mM pH 6,8 ; SDS 3,5 mM ; persulfate d'ammonium 44 mM. TEMED 8mM.

Le gel de séparation a la même composition que le gel d'alignement, sauf pour la concentration en acrylamide/bisacrylamide (8 ou 10 %) et en tampon Tris/HCl 380 mM pH 8,8.

Le tampon de migration utilisé a la composition suivante :

| | | |
|---|---|---|
| glycine | 14,4 g | |
| Tris | 3,0 g | |
| SDS | 1,0 g | |
| H₂O qsp | 1000 ml | pH 8,3. |

Les extraits à analyser ou à purifier par électrophorèse sont dilués dans un volume au moins égal du tampon de dissociation suivant : Tris/HCl 100 mM pH 6,8 ; glycérol 20 % ; SDS 70 mM, β-mercapto-éthanol 100 mM ; Bleu de bromophénol 75 µM.

Les extraits ainsi dilués sont chauffés à 100°C pendant 5 minutes.

Pour l'analyse de la composition protéique de la membrane externe, on dépose 30 à 50 µg de protéines par puits.

Pour les électrophorèses préparatives, on dépose jusqu'à 2 mg de protéines dans le puits préparatif unique.

La migration s'effectue à + 14° C pendant 5 heures à 160 V ou 14 heures à 60 V (Appareil LKB à gel vertical). Pour augmenter la résolution des protéines de la membrane externe régulées par le fer, certaines électrophorèses ont été réalisées sous une tension de 100 V pendant 16 heures. A la fin de l'électrophorèse, les protéines sont fixées et colorées pendant 30 minutes à température ambiante par du bleu de Coomassie 1,2 mM dans un mélange méthanol/acide acétique/eau (50 : 10 : 50 v/v/v). Le colorant non fixé est éliminé par plusieurs, bains successifs de méthanol/acide acétique/eau (40 : : 15 : 145 v/v/v) à 37°C. Une fois décoloré, le gel est photographié, puis séché.

Les profils protéiques des membranes externes de chaque souche peuvent être ensuite analysés par densitométrie (Densitomètre laser LKB ULTROSCAN).

### 8. Détection et analyse des anticorps anti-IROMPs

La présence d'anticorps spécifiques anti-IROMPs est recherchée par la technique ELISA en microplaques (ENGWALL et PERLMANN (10) ; ; COULTON (7)). Les antigènes couplés à la phase solide sont des fractions très enrichies en protéine proFep A ou en protéine proIut A.

La révélation des anticorps anti-IROMPs se fait au moyen d'un conjugué anti IgG de lapin (ou anti IgG de poulet) couplé à la peroxydase (Nordic). Le substrat employé est l'orthophénylène-diamine. La lecture des densités optiques s'effectue à 492 nm.
- Technique du "Western-blotting" (ou "immunobuvard-age")

Les protéines séparées par électrophorèse en gel de polycrylamide en présence de SDS sont transférées sur membrane de polyvinylidène fluorure (PVDF 0,45 um Millipore) selon la technique décrite par TOWBIN et al. (34).

Le transfert est réalisé sous 24 V pendant 1 heure avec un appareil BIOLYON en utilisant les tampons anodique et cathodique suivants :

| Tampon anodique | | Tampon cathodique | |
|---|---|---|---|
| Tris | 0,3 g | Tris | 0,3 g |
| glycine | 1,44 g | glycine | 1,44 g |
| Méthanol | 100 ml | SDS | 0,1 g |
| H₂O qsp | 500 ml | H₂O qsp | 500 ml |

Après transfert, la membrane de PVDF est saturée pendant une heure à + 37°C en tampon PBS contenant 1 % de lait écrémé.

La membrane est ensuite découpée en bandes correspondant aux pistes d'électrophorèse.

Les sérums à étudier sont dilués' dans du tampon PBS contenant 1 % de lait écrémé, puis mis en contact avec les bandes de membrane (4 ml de sérum dilué par bande).

Après incubation d'1 heure à + 37° C sous agitation douce, on effectue 3 lavages de 20 mn en tampon PBS contenant 2% de lait écrémé, à température ambiante.

Un conjugué anti-Ig G couplé à la peroxydase, dilué au 1/1000ème en PBS contenant 1 % de lait écrémé, est ajouté à raison de 3 ml par bande.

Après incubation d'1 heure à + 37°C sous agitation douce, on effectue 3 lavages de 20 mn, à température ambiante, en tampon PBS.

On ajoute alors le substrat diaminobenzidine dilué à 0,1 % en eau physiologique pH 7,15 additionné extemporanément de 0,1 % H₂O₂ 30 volumes. Des bandes de couleur marron apparaissent alors (en 5 à 20 mn) au niveau des protéines reconnues par les anticorps présents dans le sérum étudié.

La membrane est ensuite lavée en eau distillée et séchée.

### 9. Technique de préparation de l'ADN plasmidique

Les grands plasmides abrités par les souches pathogènes sont extraits selon la technique publiée par KADO et al. (16).

Les plasmides obtenus au cours des différentes étapes du clonage, du sous-clonage, et des constructions dans le vecteur d'expression sont extraits selon la technique de BIRNBOIM (BIRNBOIM et DOLY) (3). L'ADN plasmidique obtenu après les extractions préparatives réalisées avec l'une ou l'autre technique est purifié sur gradient de chlorure de césium (MANIATIS et al. (23)).

Une fois purifiés, les plasmides sont repris en tampon Tris 10 mM ; EDTA 1mM pH 8,0, de façon à avoir une concentration finale de 1 µg d'ADN/µl, et congelés à -20°C pour conservation.

### 10. Méthodes d'analyse et de modification de l'ADN

Toutes les techniques employées pour le clonage, la digestion de l'ADN, l'analyse des fragments de restriction en gel d'agarose, la modification des extrémités des fragments de restriction, l'hybridation par sonde radioactive après transfert de l'ADN sur membrane de nitrocellulose, sont celles décrites par MANIATIS (MANIATIS et al. (23)). L'ADN a été séquencé et les oligonucléotides synthétisés selon des méthodes particulières.

### 11. Séquençage de l'ADN

Les gènes à séquencer sont sous-clonés dans les vecteurs M13 mp 18 et mp 19 (YANISCH-PERRON et al. (38)) et séquencés selon la technique de terminaison de chaîne par les didésoxynucléotides (SANGER et al. (28)). Le marquage des différentes chaînes est effectué avec du ³⁵S dATP (AMERSHAM).

Le séquençage proprement dit est réalisé en utilisant les réactifs et les enzymes de kits de séquençage Amersham et Sequenase (USB). Les électrophorèses en gel de polyacrylamide en présence d'urée sont effectuées sur un appareil Sequi-Gen (BioRad).

Le traitement des données de séquence est réalisé à l'aide du logiciel Microgénie (Beckman).

### 12. Synthèse des oligonucléotides

Les différents oligonucléotides nécessaires aux constructions dans les vecteurs d'expression ou aux mutagénèses sont synthétisés selon la technique des cyano-éthyl-phosphoramidites sur un appareil Applied Systems 381-A.

Ces oligonucléotides sont utilisés directement après déprotection et précipitation à l'éthanol.

### 13. Technique de mutagénèse dirigée

La mutagénèse est réalisée selon la technique décrite par ECKSTEIN (TAYLOR et al. (33) ; NAKAMAYE et ECKSTEIN (24)) au moyen du kit de mutagénèse dirigée commercialisé par la société Amersham.

### RESULTATS

### 1. ISOLEMENT ET ANALYSE DES GENES iut A

La présence de l'opéron aérobactine a été recherchée sur les plasmides portés par les souches 15393, 15972 et 16003.

Après avoir été purifiés sur gradient de chlorure de césium, ces plasmides ont été digérés par les enzymes de restriction Bam HI, Hind III, Pvu et Sal I (Boehringer). Les différents fragments de restriction de chaque plasmide ont été séparés sur gel d'agarose à 0,8 % et transférés sur une membrane de nitrocellulose selon la technique du "Southern blot" (SOUTHERN (31)). Ces fragments ont été ensuite hybridés avec deux sondes radioactives (marquées au ³²P par déplacement de coupure) préparées à partir de fragments de restriction du plasmide pABN1 portant l'opéron aérobactine de pCol V-K30, (BINDEREIF et NEILANDS (2)). Il a été trouvé que le gène codant pour le récepteur IutA se trouve chez tous les plasmides sur un fragment de restriction Bam HI-Bam HI de 6,6 kb. Ces résultats confirment les travaux des auteurs qui ont sous-cloné ce gène à partir d'un fragment équivalent de pCol V-K30 (KRONE et al. (17)).

### - Clonage des fragments d'ADN plasmidique portant le gène iut A

Les plasmides des souches 15393, 15972 et 16003 ont été digérés par l'enzyme de restriction Bam HI. Après électrophorèse en gel d'agarose à 0,8 %, la bande de gel contenant le fragment de taille 6,6 kb de chaque plasmide a été découpée et l'ADN a été électroélué.

Les trois fragments Bam HI-Bam HI 6,6 kb ont été ligués séparément dans le vecteur paT 153 digéré par Bam HI. Les trois mélanges de ligation ont servi à transformer des bactéries HB 101 compétentes (Tableau IV).

L'ADN des clones ampicilline résistants, tétracycline sensibles a été extrait (par la technique de BIRNBOIM et DOLY) et digéré par Bam HI pour vérifier la taille de l'insert. Les clones ayant intégré le fragment de 6,6 kb ont été sélectionnés sur la base de leur sensibilité à la cloacine DF 13.

Pour chaque plasmide de départ, un clone sensible à la cloacine a été retenu pour les analyses et les constructions ultérieures. Il s'agit des clones :

| | |
|---|---|
| Souche 15393 | clone HB 101 p 5-15 |
| Souche 15972 | clone HB 101 p P-13 |
| Souche 16003 | clone HB 101 p 4-18 |

### - Analyse des clones

### Etude de l'expression du récepteur Iut A

Le fait d'avoir pu sélectionner les clones recherchés au moyen d'un test de sensibilité à la cloacine montre qu'il y a une expression du récepteur à la cloacine et à l'aérobactine. L'expression du gène iut A porté par le fragment Bam HI-Bam HI 6,6 kb est probablement sous la dépendance d'un promoteur faible situé sur ce fragment (KRONE et al. (17)) et non dépendante de la concentration en fer comme l'est le promoteur principal de l'opéron aérobactine. En effet, la sensibilité à la cloacine est mise en évidence par culture sur gélose LB-ampicilline riche en fer.

Pour étudier le niveau d'expression du récepteur Iut A, les différents clones ont été cultivés en milieu LB-ampicilline (pendant une nuit à + 37°C) en présence ou non d'ovotransferrine (500 µg/ml).

Les protéines de la membrane externe des trois clones' ont été extraites et analysées sur gel de polyacrylamide en présence de SDS.

Quelles qu'aient été les conditions de culture, il n'a pas été possible d'observer l'expression d'une protéine surnuméraire de 76 kDa chez les clones sensibles à la cloacine.

### - Cartes de restriction

L'ADN plasmidique des clones 4-18, 5-15 et P-13 a été extrait en grande quantité et purifié sur gradient de chlorure de césium.

Les cartes de restriction des trois fragments Bam HI-Bam HI 6,6 kb clonés ont été établies avec les enzymes Bgl II, Bst E II, Cla I, Eco RI, Kpn I, Pst I, Pvu II et Sma I.

Ces trois cartes sont rigoureusement identiques entre elles et correspondent à la carte de restriction du fragment Bam HI-Bam HI 6,6 kb de l'opéron aérobactine de p ColV-K30 déduite des cartes publiées par BINDEREIF et NEILANDS (1,2) et KRONE et collaborateurs (17).

Ces quatre cartes sont présentées sur le Tableau V.

### - Séquençage des gènes iut A

Lors de la comparaison des cartes de restriction des trois fragments Bam HI-Bam HI 6,6 kb avec la carte de restriction déduite de la séquence du gène iut A (KRONE et al. (18)), il est apparu que le gène iut A était entièrement localisé sur un fragment de restriction Bst E II-Bst E II de 3,2 kb (Tableau VI).

Ce fragment a été isolé par électroélution, religué sur lui-même avec la ligase de phage T4 (Boehringer) et digéré avec plusieurs systèmes d'enzymes de restriction. Les différents fragments ainsi obtenus (taille de 150 à 600 pb) ont été isolés par Geneclean (Bio 101) et sous-clonés dans les vecteurs M13 mp 18 et mp 19 digérés au préalable avec les enzymes appropriées. La séquence de chaque sous-clone a ensuite été déterminée selon la technique de SANGER en utilisant les kits de séquençage Amersham et Sequenase (USB).

Seul le fragment Bst E II-Bst E II 3,2 kb du clone P-13 a été séquencé en totalité. Les deux autres gènes iut A ont été séquencés entre le site Bgl II (1) et le site Eco RV (2396).

La région Bst E II-Bgl II du clone P-13 a été comparée avec la séquence du gène iuc D située juste en amont de iut A (HERRERO et al. (14)) et la région Pvu II-Bst E II de ce clone a été comparée avec la séquence de l'élément IS1 (OHTSUBO et OHTSUBO (25)).

Ces comparaisons ainsi que les comparaisons des séquences des trois gènes iut A avec la séquence du gène iut A de pColV-K30 sont présentées sur la Tableau VII.

L'analyse de ces quatre séquences a révélé que le gène iut A était extrêmement conservé au niveau moléculaire. A une ou deux bases près, les trois gènes iut A isolés de souches E. coli d'origine animale sont identiques entre eux. Les différences observées vis-à-vis de la séquence publiée par KRONE et al. (18) sont minimes.

Les trois gènes iut A étudiés ont 99,77 % d'homologie avec le gène iut A de pColV-K30.

Les régions où des différences ont été constatées sont présentées sur le Tableau VIII. (Les chiffres renvoient à la position des bases dans les séquences présentées sur le Tableau VII).

Deux régions importantes sont strictement conservées : la séquence codant pour le peptide signal et la séquence consensus ("Boîte Ton B") typique des récepteurs protéiques de la membrane externe dont la fonction dépend de Ton B.

Il a été mis en évidence, chez chacun des trois gènes séquencés, l'existence de quatre insertions par rapport au gène iut A de ColV-K30. Ces insertions provoquent des changements limités du cadre de lecture. La structure primaire des protéines Iut A codées par les plasmides isolés des souches utilisées est de ce fait un peu plus grande (+ 8 acides aminés) que celle de la protéine Iut A de p ColV-K30. La séquence des gènes iut A isolés des souches utilisées code pour un polypeptide de 733 acides aminés comprenant un peptide signal de 25 acides aminés identique à celui du polypeptide Iut A de la souche ColV-K30. La masse calculée de la protéine mature est de 78097 daltons, ce qui est légèrement différent de la taille observée sur gel (76 kDa). Les changements dans la structure primaire ne sont cependant pas assez importants pour modifier la structure secondaire et le profil d'hydrophilicité.

### 2. EXPRESSION DES GENES iut A ET fep A CLONES

### - Caractéristiques du vecteur utilisé

Le vecteur d'expression employé est le plasmide GTI 001 construit par le laboratoire de génie génétique de l'Institut Mérieux.

Les gènes qu'il porte et sa carte de restriction sont présentés dans le Tableau IX.

Ce plasmide peut être construit comme suit :

Le plasmide pBRTac, constitué par pBR322 (Bolivar F. et al., Gene 2, 95-113 (1977)) propageant entre HindIII et BamHI le promoteur Tac (Ammann E. et al, Gene 25, 167 (1983)), a son site XhoI détruit par la polymérase de Kleenow (ou "la kleenow") pour donner le plasmide pBRTacX⁻. Ce plasmide est digéré par NcoI, traité par la kleenow, puis digéré par AvaI, et son plus petit fragment est ligué au fragment de pMC9 (Casadaban M.J. et al., Journal of Bacteriology, 143, 971-980 (1980)) digéré par MstII, traité par la kleenow, puis digéré par AvaI portant le gène Lac i et l'origine de réplication de pBR322.

Le plasmide résultant (nommé pBRLaciX⁻) est digéré par HindIII, traité par la kleenow puis digéré par PstI, et le fragment de 2350 paires de bases (ou "pb") est ligué au fragment de 2300 pb de pBRTac digéré par EcoRI, puis traité par la kleenow, puis digéré par PstI, créant ainsi le plasmide pBRTaci. Le fragment de 4406 pb de ce dernier, obtenu par digestion avec EcoRI et PstI est ligué avec un fragment de 1688 pb digéré par EcoRI et PstI dérivé de séquences de pBR322 et portant le gène de résistance à la tétracycline de pBR322.

Le plasmide obtenu est nommé pBRTaciTet. L'origine de réplication de ce dernier est séparée par digestion par BamHI, et le fragment de 2096 pb restant est ligué avec le fragment de 2033 pb de pAT153 (Twigg A.J. & Sherratt, D. Nature, 283, 216-218 (1980)) digéré par XhoI, créant ainsi le plasmide pATTaciOri.

Ce plasmide est digéré par EcoRI, puis traité à la kleenow, puis digéré par Aval et le fragment de 2704 pb est ligué avec un fragment portant le promoteur Pr et son répresseur thermosensible CI857 du bactériophage Lambda de 3076 pb issu de pCQV2 (Queen c. et al., J. Mol. Appl. Genet. 2, 1-10 (1983)) par digestion par PstI, traitement à la nucléase Mung Bean, et digestion partielle par AvaI. Le plasmide résultant est nommé pGTI001.

L'origine de réplication de ce plasmide est sous le contrôle du promoteur tac, ce qui permet de maîtriser le nombre de copies en cultivant les bactéries dans un milieu contenant une concentration plus ou moins grande d'IPTG (Inducteur du gène lac i).

Le gène à exprimer est placé sous le contrôle du promoteur fort "Pr" du phage CI 857 (dont le répresseur est thermosensible). L'ATG du gène à exprimer est remplacé par l'ATG du gène cro. Cet ATG est créé par une digestion partielle Bam HI de p GTI 001, suivie d'une digestion par la Mung Bean nucléase afin d'obtenir une extrémité ATG à bout franc.

Le gène à exprimer est alors inséré en phase (à partir du codon N° 2) entre l'extrémité ATG à bout franc et le site XhoI. Un signal de terminaison de la transcription, placé juste en aval de ce site XhoI, évite la formation d'ARNs messagers trop longs.

D'autres plasmides de ce genre susceptibles d'exprimer le gène iut A (ou fepA), sont faciles à obtenir ou à construire et l'on connaît de tels plasmides dans lesquels le gène à exprimer est contrôlé par un promoteur dont le represseur est thermosensible.

### - Construction du vecteur d'expression du gène iut A

Les gènes iut A isolés des souches 15972 et 16003 ont été clonés avec leur séquence signal au niveau du site d'expression Bam HI (899) de p GTI 001.

Pour obtenir une extrémité 5' à bout franc commençant au deuxième acide aminé de la séquence signal (en l'occurrence la méthionine), un oligonucléotide de synthèse double brin a été utilisé pour remplacer la région comprise entre l'ATG d'initiation et le site Acc I unique situé en 5' de la séquence codante. La séquence de cet oligonucléotide est présentée sur le Tableau X.

Les deux brins complémentaires de cet oligonucléotide ont été synthétisés et la forme double brin a été obtenue par chauffage à 90°C en tampon NaCl 50 mM, Tris 10 mM, MgCl 10 mM pH 7,5, d'un mélange équimolaire des deux simples brins et refroidissement lent à température ambiante.

La stratégie suivie pour insérer le gène iut A de la souche 15972 (clone p P-13) est présentée sur le Tableau XI.

Le rendement n'ayant pas été très satisfaisant, une stratégie modifiée a été adoptée pour la mise en phase du gène iut A de la souche 16003. Cette nouvelle stratégie a fait appel à un sous-clonage de la région Eco RI-Eco RI de la construction intermédiaire (Etape 4, Tableau XII) dans le vecteur pSB 118. Ce vecteur est un dérivé pUC 18. Il possède un "polylinker" encadré par deux sites Eco RI. Le sous-clonage du fragment Eco RI-Eco RI dans ce vecteur a donc permis de réaliser la mise en phase du gène iut A en évitant les digestions partielles (Tableau XII).

Les cartes de restriction des plasmides d'expression GTI P-2 (gène iut A de la souche 15972 )et GTI B-5 (gène iut A de la souche 16003) ainsi obtenus sont présentées sur le Tableau XIII.

Les clones obtenus après nouvelle ligation avec l'oligonucléotide "iut" double brin ont été sélectionnés sur la base de la conservation du site Acc I du gène iut A et de la disparition du site Bam HI de p GTI 001. Tous les clones présentant ce profil de restriction ont été ensuite contrôlés au niveau de l'expression de la protéine Iut A.

### - Contrôle de l'expression d'Iut A

### Contrôle qualitatif

Les clones sélectionnés ont été cultivés en milieu M9 SP tétracycline en présence d'IPTG 0,4 mM à 32°C (début d'induction). La sensibilité de ces clones vis-à-vis de la cloacine a été recherchée par la technique décrite précédemment.

2 clones sur 25 ont été positifs pour les constructions GTI-iut 15972

3 clones sur 6 ont été positifs pour les constructions GTI-iut 16003

Les clones sensibles à la cloacine sont cultivés dans 50 ml de milieu M9 SP tétracycline contenant de l'IPTG 0,4 mM.

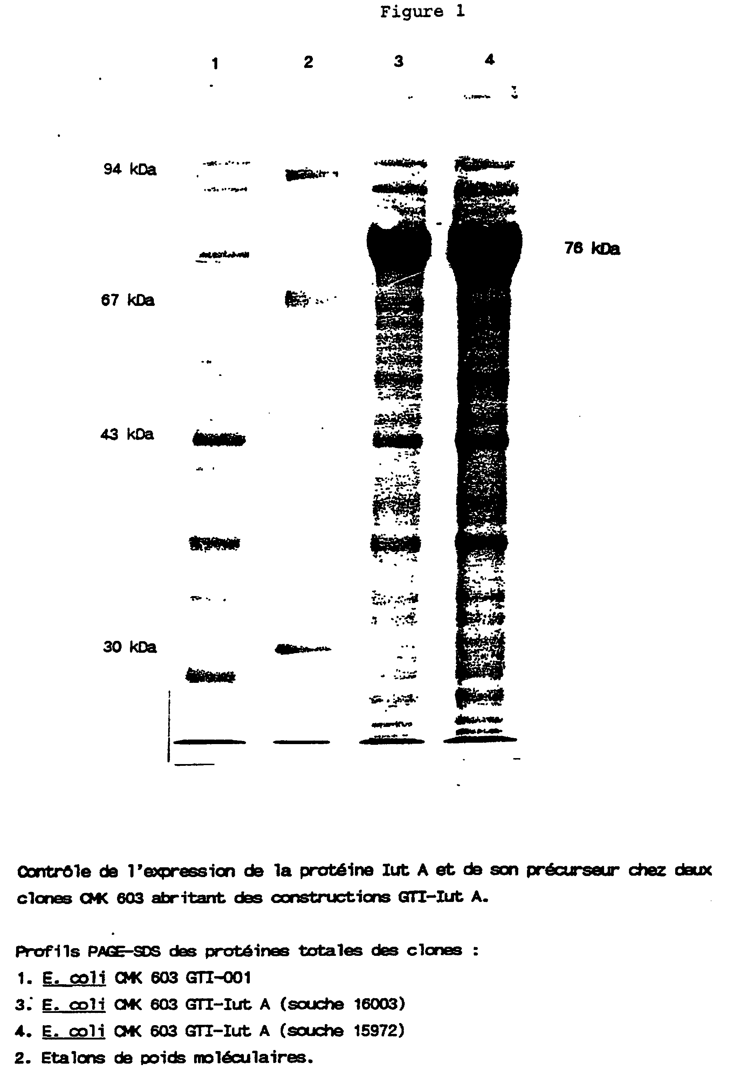

La culture est faite à la température de 30°C jusqu'à ce que la densité optique atteigne la valeur de 1. L'induction de l'expression du gène iut A est alors réalisée en continuant la culture pendant 4 heures à + 42°C. Les bactéries sont centrifugées et leurs protéines totales sont analysées par électrophorèse en gel de polyacrylamide-SDS. Ceci permet d'apprécier directement l'importance de la production de la protéine Iut A (figure 1). L'analyse des protéines du clone CMK 603 GTI P-2 a révélé que la protéine Iut A et son précurseur représentaient, après induction, 25 % des protéines totales de la bactérie. La protéine Iut A seule représente 30 % des protéines de la membrane externe.

### - Construction du vecteur d'expression du gène fep A

### Caractéristiques du clone initial p MS 101

Suivant les résultats de CODERRE et EARHART (6) qui indiquaient que le gène fep A était situé sur un fragment Bam HI-Bam HI de 6,3 kb du plasmide pMS 101 construit par LAIRD et YOUNG (19), ce fragment a été sous-cloné dans le vecteur pBR 322 digéré par Bam HI. La carte de restriction du plasmide obtenu (F-1) s'est révélée semblable à celle du plasmide pITS 1 (FLEMING et al. (11)) (Tableau XIV).

La publication de la séquence de fep A (LUNDRIGAN et KADNER) (22) a permis de situer précisément ce gène sur le fragment de restriction Ssp I-Stu I 2530 pb du plasmide F-1.

La stratégie suivante a été utilisée pour insérer le gène fep A dans p GTI 001.

Une mutagénèse dirigée a été réalisée en région 5' terminale de la région codante pour transformer la séquence : 5' ATGAACAAG 3' en un site de restriction Hpa I

MET    ASN    LYS

—

G T T A A C A A G

Ce site est coupé en bouts francs de la façon suivante GTT AA C. Cela permet une ligation directe du gène fep A avec l'extrémité ATG créée dans p GTI 001.

La mutagénèse a été effectuée selon la technique d'ECKSTEIN à partir d'un oligonucléotide (Tableau XV), après sous-clonage du fragment Ssp I-Eco RI 800 pb dans la forme réplicative du phage M13 mp 19.

Le fragment muté a été entièrement séquencé pour vérifier que la séquence n'avait pas été modifiée ailleurs qu'au site visé.

Les modalités d'intégration du gène fep A sont résumées sur les Tableaux XVI et XVII.

Les différents clones obtenus après ligation du fragment HpaI-XhoI 2350 pb dans le plasmide GTI 001 ont été sélectionnés sur la présence d'un fragment Eco RI- Eco RI de 1700 pb.

### - Contrôle de l'expression de Fep A

### Contrôle qualitatif

Le mélange de ligation entre le fragment Hpa I- Xho I 2350 pb et le plasmide GTI 001 a servi à transformer des bactéries compétentes RWB 18. Cette souche étant fep A, elle est résistante à la colicine B.

Les clones ayant la carte de restriction recherchée (Tableau XVII) ont été testés sur leur sensibilité à la colicine B.

Un clone (RWB 18 GTI F-12) s'est révélé sensible à l'action de la colicine B.

### Contrôle quantitatif

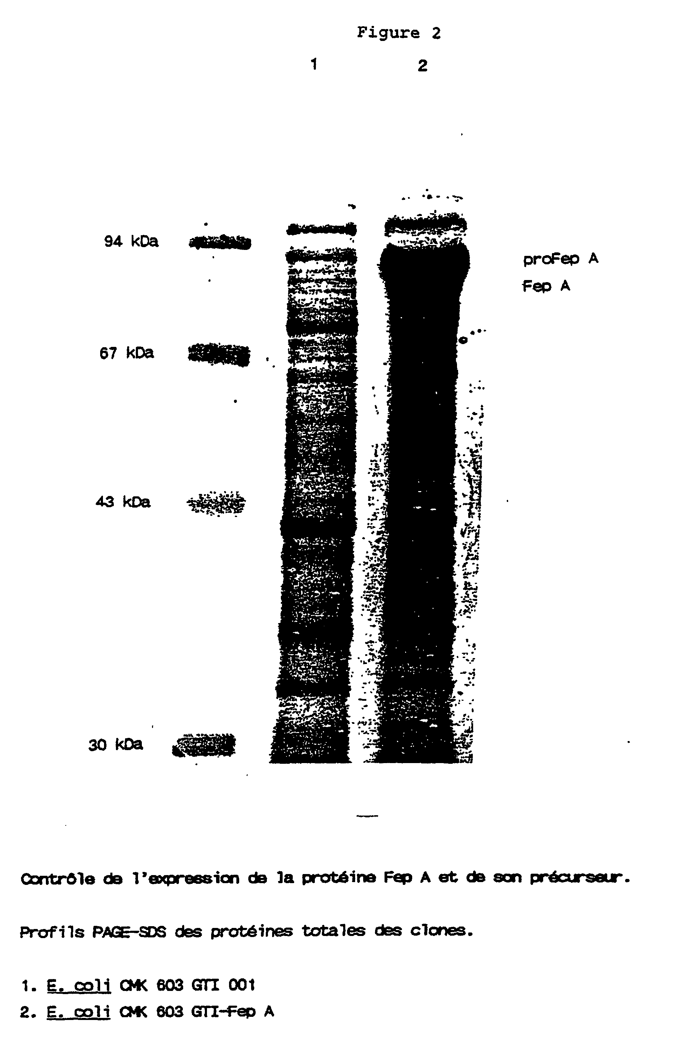

L'expression de la protéine Fep A et de son précurseur a été analysée sur gel de polyacrylamide-SDS (figure *2).* Le clone CMK 603 GTI F-12 exprime Fep A et son précurseur en très grosse quantité (20 % des protéines totales).

La protéine Fep A représente 32 % des protéines de la membrane externe.

### - Etude physiologique et morphologique des clones exprimant Iut A et Fep A

### Aptitude à la culture

L'aptitude à la culture des clones obtenus a été testée à différentes températures de culture en milieu LB tétracycline IPTG.

Ensemencés en nappe sur gélose LB tétracycline IPTG 0,1 mM, tous les clones forment des tapis bactériens sensibles aux bactériocines lorsqu'on les cultive à des températures comprises entre 30°C et 34°C.

Au-delà de 34°C, les tapis bactériens ne se forment plus. Ce phénomène a également été observé en milieu LB liquide.

La sensibilité aux bactériocines est retrouvée pour des concentrations en IPTG aussi basses que 0,05 mM et à la température de 30°C. Il existe donc un certain niveau d'expression des gènes iut A et fep A, en l'absence d'induction du vecteur p GTI 001.

### Etude morphologique

Les différents clones subissent des modifications morphologiques consécutives à la surexpression d'Iut A et de Fep A.

Les bactéries, observées au microscope optique à contraste de phase après induction à + 42°C pendant 4 heures, présentent un allongement important (jusqu'à dix fois la longueur moyenne d'une Escherichia coli K12 normale). Cependant, la caractéristique la plus frappante est la présence d'une à plusieurs inclusions intracytoplasmiques à l'intérieur de chaque corps bactérien.

Les inclusions observées au microscope optique sont retrouvées lors de l'observation au microscope électronique après coloration négative de coupes de bactéries cultivées à + 42°C pendant 4 heures.

Ces inclusions sont périphériques et adjacentes à la face interne de la membrane cytoplasmique.

### 3. PROPRIETES IMMUNOLOGIQUES DES PROTEINES Iut A ET Fep A

### - Immunogénicité de Iut A et de Fep A

Les protéines Iut A, extraites des membranes externes des souches E. coli 15022, 15393, 16003 et de la souche recombinante Escherichia coli CMK 603 GTI P-2, ont été isolées par gels de polyacrylamide préparatifs et injectées à des lapins selon le protocole décrit précédemment.

L'évolution du titre des anticorps anti-Iut A produits par chaque lapin a été évaluée par la technique ELISA en prenant comme antigène une solution de "granules" (protéine proIut A précipitée) extraits d'une culture de la souche E. coli CMK 603 GTI P-2.

Le sérum positif de référence utilisé est un sérum de lapin anti-protéine Iut A d'E. coli ColV-K30 qui a été fourni par B. OUDEGA.

Le même protocole a été suivi pour les protéines Fep A extraites des membranes des souches E. coli 15022 et E. coli RWB 18 GTI F12.

Dans tous les cas, les lapins ont réagi à l'injection des protéines Iut A et Fep A en produisant un titre élevé d'anticorps spécifiquement dirigés contre ces protéines.

### - Propriétés antigéniques des protéines Iut A et Fep A

La spécificité des différents anticorps obtenus a été étudiée vis-à-vis de plusieurs préparations de membranes externes (souches E. coli 15022, 15393, CMK 603 GTI P-2, CMK 603 GTI B-5 et RWB 18 GTI F-12). Cette étude a été réalisée par la technique du "Western blot".

Les quatre sérums anti-Iut A produits, de même que le sérum anti-Iut A colV-K30 de référence, reconnaissent spécifiquement une protéine de 76 kDa dans toutes les préparations de membranes externes des souches exprimant le système aérobactine. Quelle que soit la protéine Iut A (sauvage ou recombinante) ayant servi à leur induction, les anticorps d'un même sérum reconnaissent spécifiquement la protéine Iut A exprimée par les souches E. coli d'origine aviaire et bovine et les deux protéines Iut A produites par les souches recombinantes E. coli CMK 603 GTI P-2 et CMK 603 GTI B-5.

Le précurseur de la protéine Iut A, la protéine proIut A est, elle aussi, reconnue spécifiquement par tous les sérums anti-Iut A. (Immunoblots réalisés avec les "granules" purifiés produits par les souches CMK 603 GTI P-2 et CMK 603 GTI B-5).

La protéine Fep A n'est pas reconnue par les sérums anti-Iut A et, inversement, les sérums anti-Fep A ne reconnaissent pas les protéines Iut A.

Le clonage des gènes iut A et fep A dans le vecteur d'expression GTI 001 permet de produire en grande quantité les protéines Iut A et Fep A ainsi que leurs précurseurs respectifs. Les protéines Iut A ou Fep A et leurs précurseurs synthétisés à la suite de l'induction de la transcription par culture des bactéries à 42°C, s'accumulent rapidement sous forme d'inclusions cytoplasmiques (granules) de grande taille, visibles au microscope optique à contraste de phase. L'observation au microscope électronique de coupes de bactéries induites révèle que ces granules sont étroitement accolés à la face interne de la membrane cytoplasmique.

Il faut noter l'importance du niveau d'expression des précurseurs d'Iut A et de Fep A (25 % des protéines totales en moyenne dans des conditions non optimisées). Les protéines matures représentent jusqu'à 35 % du contenu protéique de la membrane externe. On peut considérer ce pourcentage comme la limite supérieure de l'intégration de ce type de protéine dans la membrane externe. A titre de comparaison, les protéines Iut A et Fep A exprimées par la souche sauvage Escherichia coli 15022 représentent ensemble 30 % des protéines de la membrane externe. Il apparaît donc que l'expression totale des protéines Iut A et Fep A, et de leurs précurseurs, par les souches recombinantes conformes à l'invention est très supérieure à l'expression naturelle.

La mise en évidence d'une sensibilité à la cloacine DF 13 chez les clones exprimant la protéines Iut A, et d'une sensibilité à la colicine B chez ceux qui expriment la protéine Fep A démontre que la synthèse et l'intégration de ces protéines dans la membrane externe se déroulent normalement.

L'identité des protéines obtenues par recombinaison génétique avec les protéines sauvages est également démontrée par la reconnaissance de ces protéines par des anticorps dirigés contre les protéines Iut A et Fep A naturelles. On notera que ces anticorps reconnaissent aussi, avec la même spécificité, les précurseurs proIut A et proFep A précipités dans les inclusions intracytoplasmiques.

Les anticorps induits par les protéines Iut A et Fep A obtenues par recombinaison génétique reconnaissent, de la même façon, spécifiquement les protéines Iut A et Fep A exprimées par différentes souches d'Escherichia coli pathogènes.

La surexpression des récepteurs Iut A et Fep A par clonage de leurs gènes sur un vecteur d'expression, permet d'obtenir, dans un milieu riche en fer, des protéines de membrane externe fonctionnellement et antigéniquement identiques aux protéines exprimées par les bactéries pathogènes au cours de leur multiplication in vivo.

La synthèse des protéines Iut A et Fep A par recombinaison génétique présente donc de nombreux avantages :
- elle permet d'obtenir les protéines Iut A et Fep A en très grande quantité, tout en s'affranchissant de la régulation par le fer,
- les protéines synthétisées sont fonctionnellement et antigéniquement identiques aux protéines exprimées par les bactéries pathogènes au cours de leur multiplication in vivo et induisent la formation d'anticorps,
- utilisées, à titre de principe actif, dans un vaccin, elles induisent la formation d'anticorps qui empêchent la reconnaissance spécifique, par les protéines de membrane régulées par le fer, des sidérophores, arrêtant par là même l'alimentation en fer et bloquant leur multiplication chez l'hôte ; elles permettent donc, à ce titre, la préparation de vaccins très utiles pour prévenir ou lutter contre les infections y compris septicémies.

Les vaccins peuvent aussi être préparés simplement à partir des clones recombinants inactivés ou à partir de fragments de membrane obtenus par lyse des clones recombinants suivi d'une purification, selon les techniques usuelles pour la fabrication de vaccins à base d'antigènes de surface ou de paroi.

### IV. PREPARATION D'IROMPS PAR CULTURE EN MILIEU RESTREINT EN FER.

1) Souche : E. coli 078 référence 15022 : Origine poulet
2) Culture :
   - Milieu :
      Milieu ST + succinate (Simon E. H. et Tesmann (1963) Proc. Natl. Acad. Sci USA 50, 526-532)
      . additionné de lactoferrine (250 µg/ml) pour obtenir un milieu carencé en fer,
      . ou additionné de FeCl₃, 6H₂O (40 µM) : pour un milieu riche en fer.
   - Culture :
      - 3 passages de la souche en milieu riche en fer suivis d'un passage d'adaptation en milieu carencé avant culture finale en milieu carencé
      - parallèlement on effectue une culture de la même souche en milieu riche en fer,
      - les cultures sont menées à 37°C pendant 24 heures.
3) Analyse :
   En fin de culture, dans chaque milieu, on procède aux opérations suivantes :
   - Récolte par centrifugation,
   - Récolte du culot en Tris HCl 0,2 M pH 8 et ultrasonication,
   - Centrifugation (5000 g 30 minutes),
   - Surnageant recentrifugé (100 000 g, 1 heure),
   - Reprise du culot en Tris HCl (50 mM, pH 8), MgCl₂ (10 mM), EDTA 1 mM, Triton x 100 (2 %) et agitation 20 minutes à 37°C,
   - Centrifugation 1 heure à 100 000 g puis réextraction du culot,
   - Lavage du culot en eau déminéralisée,
   - Analyse du culot en gel de polyacrylamide (PAGE SDS) en conditions dénaturantes (mercaptoéthanol, SDS).
4) Résultat :
   - Membranes des bactéries cultivées en présence de lactoferrine : présence de deux protéines, en quantité importante, de poids moléculaire apparent 80000 da (récepteur de l'entérobactine, Fep A) et 76000 da (récepteur de l'aérobactine, Iut A),
   - Membranes des bactéries cultivées en milieu riche en fer : absence de bande de 76000 et 80000 da.

### V - PREPARATION DE VACCINS.

De préférence, les principes actifs selon l'invention seront associés, dans les vaccins, à des préparations antigéniques classiques dans les vaccins anti-bactériens humains ou vétérinaires connus, et ceci notamment, dans le cas où l'on utilise les protéines purifiées.

Ces vaccins peuvent présenter les principes actifs selon l'invention dans des véhicules liquides usuels pour l'administration par voie parentérale. Ils peuvent comprendre des adjuvants classiques, par exemple huileux.

**TABLEAU I**

| Souches pathogènes | | Sérotype | Animal d'Origine | Référence |
|---|---|---|---|---|
| E. coli | 15 022 | O 78 | Poulet | Souchothèque RM |
| " | 15 393 | O 86 | Veau | |
| " | 15 972 | O 2 | Poulet | " |
| " | 16 000 | O 138 | Veau | " |
| " | 16 003 | O 138 | Veau | " |
| E. coli | KM 576 | (p ColV-K30) | Homme | B. OUDEGA |

| Souches hôtes E. coli K12 | génotype | | origine ou référence |
|---|---|---|---|
| . C 600 | F- | thr, thi, leu, lac Y. fhu A, SUP E | (MANIATIS et al.) |
| . HB 101 | F- | hsd (r-B, m-B),recA, ara, pro, lacY, gal, rps, xyl, mtl, supE. | (MANIATIS et al. ) |
| . RWB 18 | F- | thi, proG. leu, trp, entA, fepA | (HOLLIFIED et NETLANDS (14) |
| . CMK 603 | F⁺ | thr, thi, leu, supE, recBC, fhu A, lac Y, r-B, m+ | Institut Mérieux |
| .15525 | F- | | Souchothèque RM |

| Souches productrices de bactériocines | |
|---|---|
| . E. coli 1300 | Productrice de colicine B R. PORTALIER |
| . Enterobacter clocae DF 13 S458 | Productrice de cloacine DF13 B. OUDEGA |

**TABLEAU II**

| Nom | Taille (paires de bases) | Caractéristiques | Référence ou origine |
|---|---|---|---|
| pBR 322 | 4363 | Amp^{r} Tet^{r} Vecteur de clonage | F. BOLIVAR |
| pAT 153 | 3600 | Amp^{r} Tet^{r} Vecteur de clonage | A. TWIGG |
| pSB 118 | 2692 | Amp^{r} Dérivé de pUC18 Vecteur de clonage | P. STRAGIER I^{t}.Pasteur Paris |
| pGTI 001 | 5780 | Tet^{r} Vecteur d'expression | P. BRUNEAU I^{t} Mérieux |
| pABN 1 | 18300 | Amp^{r} Vecteur p Plac + fragment Hind III 16,3 kb de ColV-K30 portant l'opéron aérobactine | BINDEREIF et NEILANDS (2) |
| pMS 101 | 15300 | Amp^{r} Vecteur pBR 322 + fragment Hind III de 11 kb du chromosome de E. coli portant les gènes entD, fepA, fes et entF | LAIRD et YOUNG (19) |

**TABLEAU III**

| Jour 0 | Jour 14 | Jour 28 | Jour 35 |
|---|---|---|---|
| injection intradermique | injection intramusculaire | injection intramusculaire | injection intramusculaire |
| 125µg Prise de sang 5 ml | 125µg | 125µg Prise de sang 10 ml | 250µg |

| Jour 42 | Jour 49 | Jour 56 | |
|---|---|---|---|
| injection intramusculaire | injection intramusculaire | | |
| 250µg | 250µg | | |
| Prise de sang 50 ml | | Prise de sang 60 ml | |

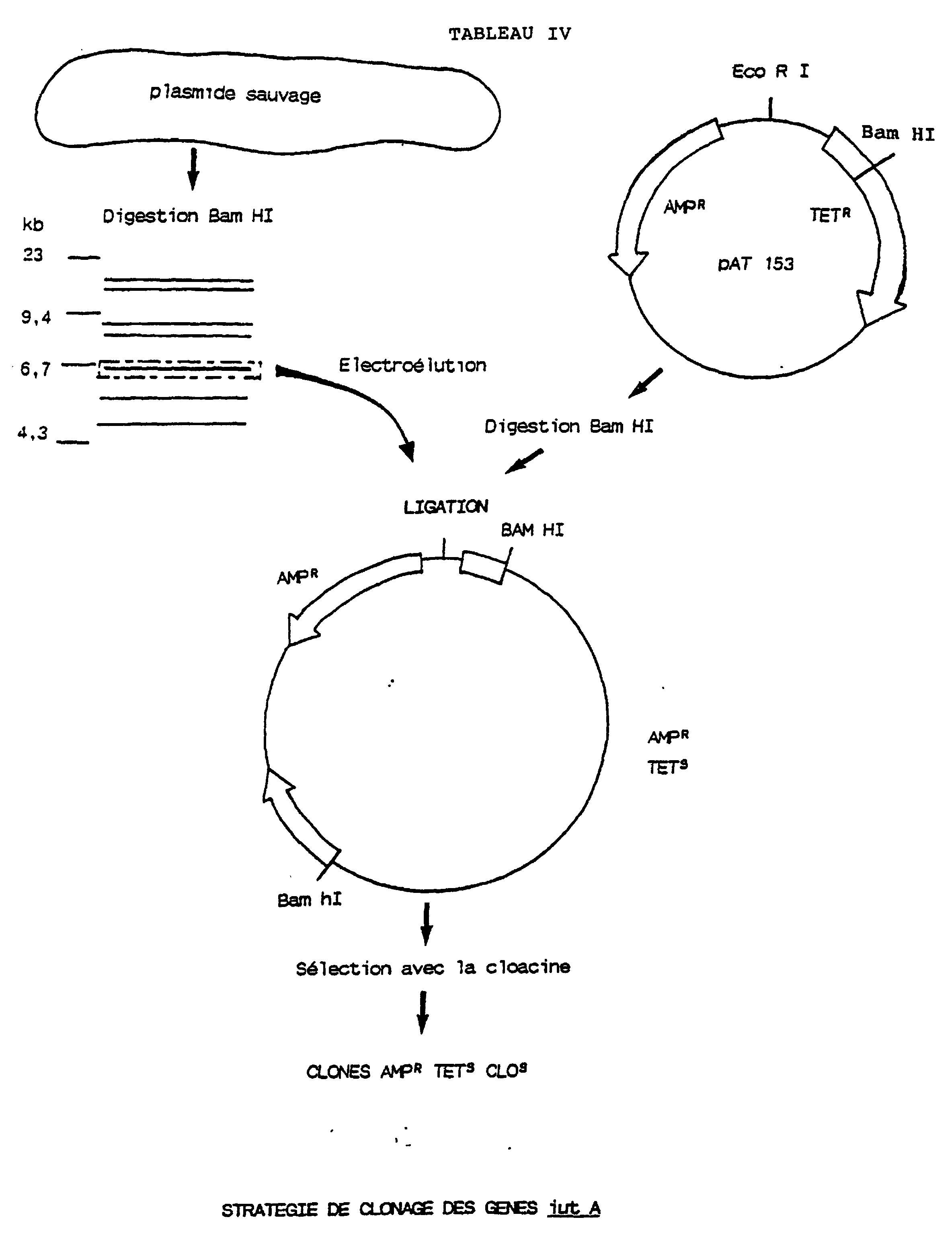

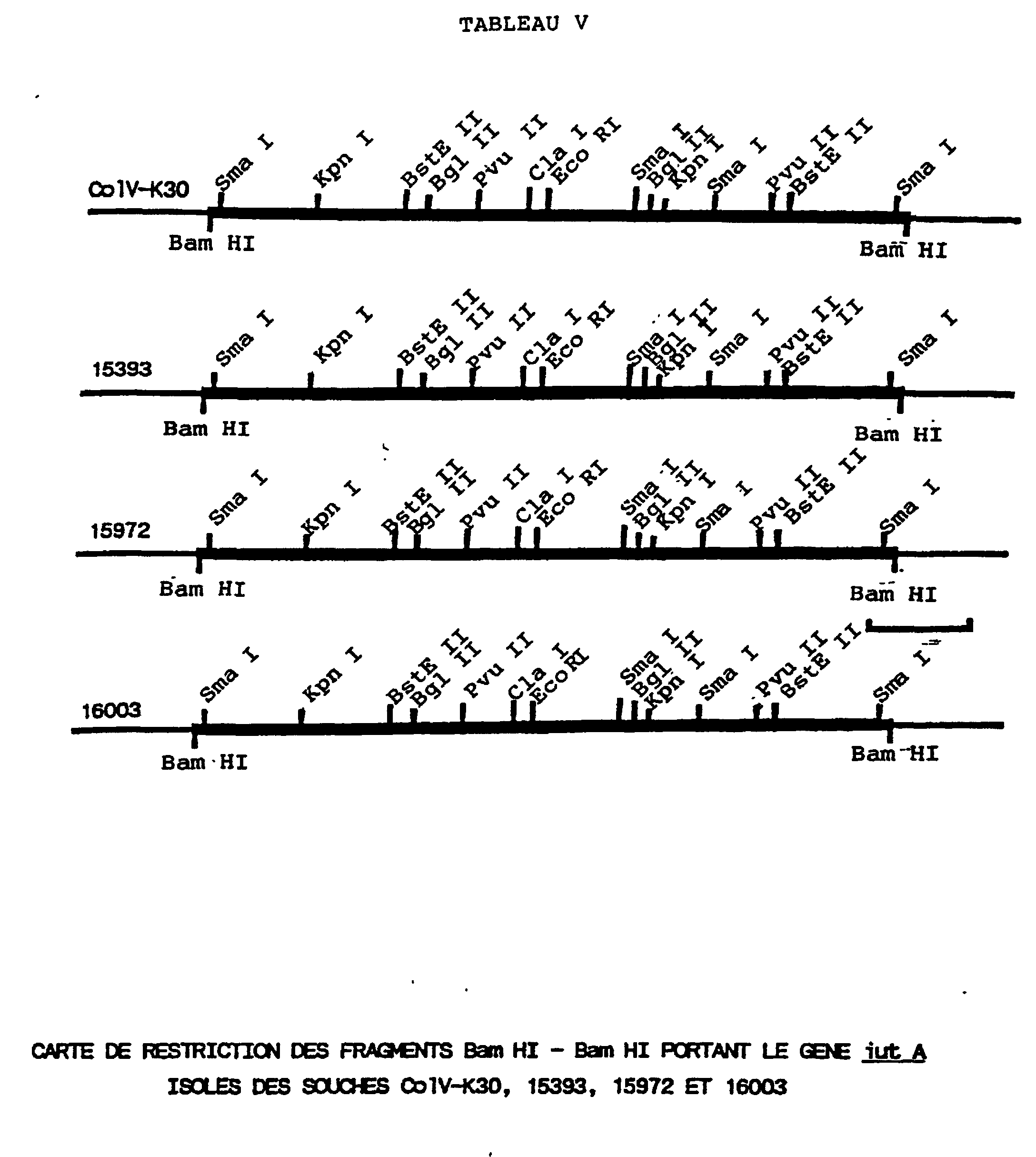

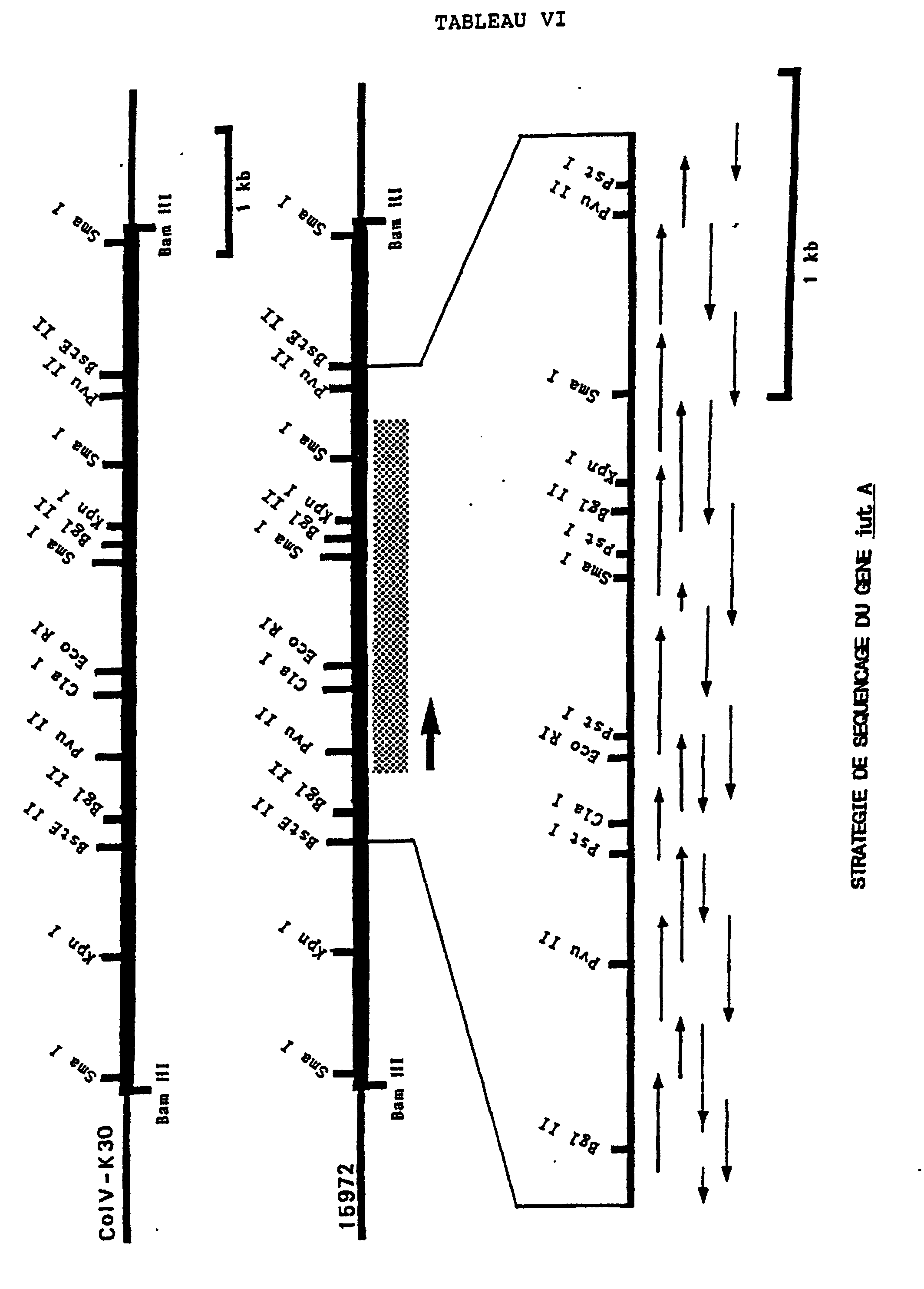

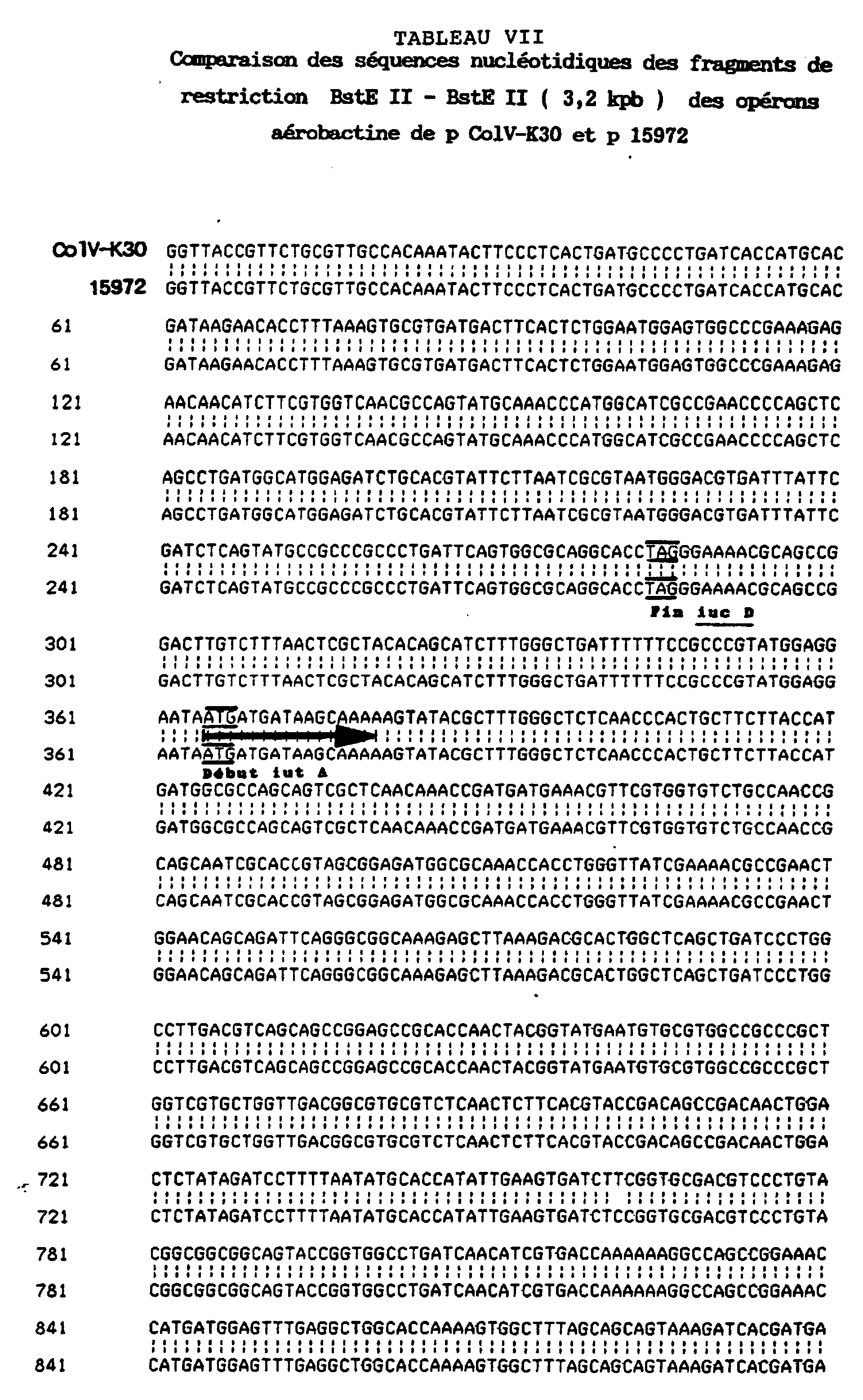

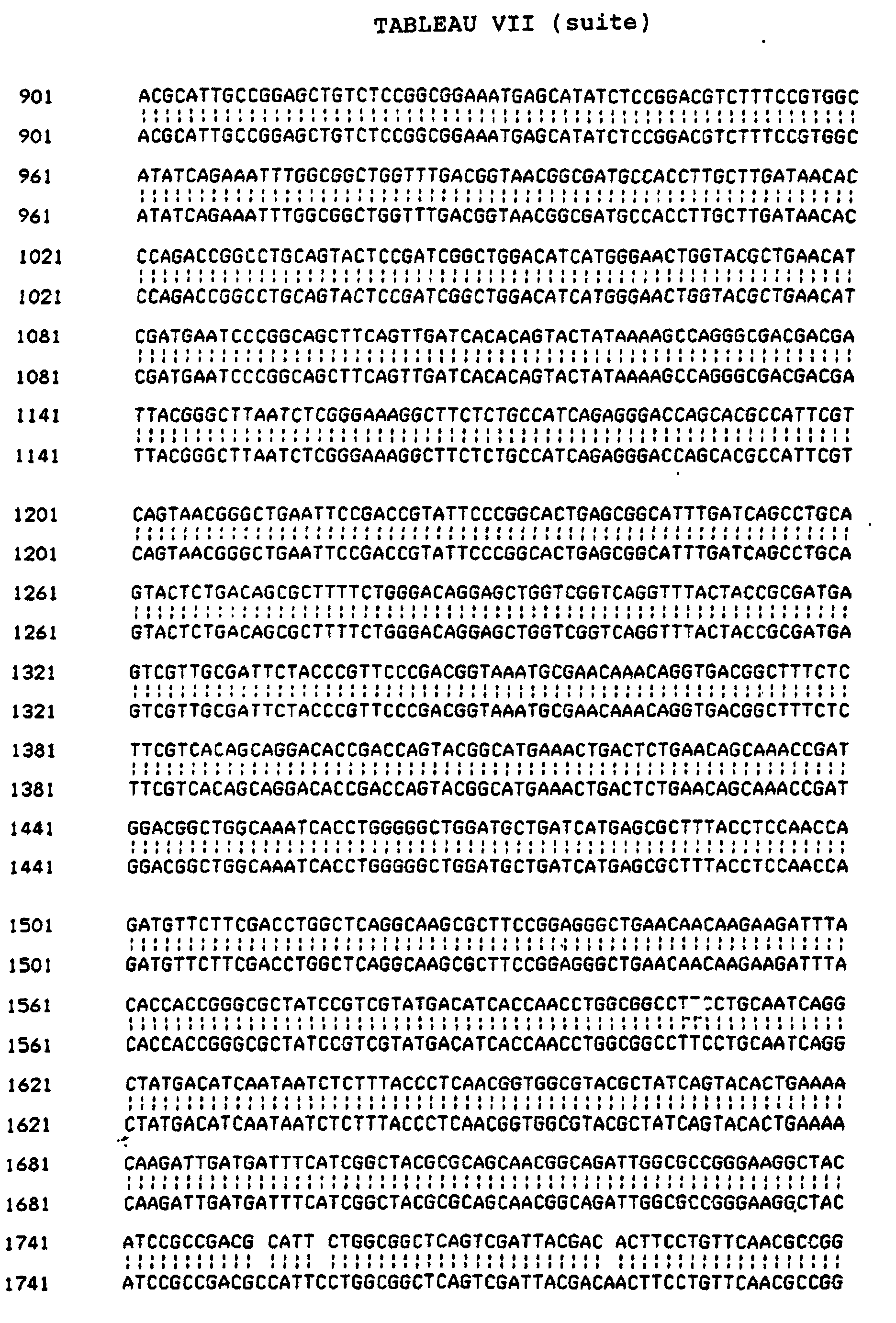

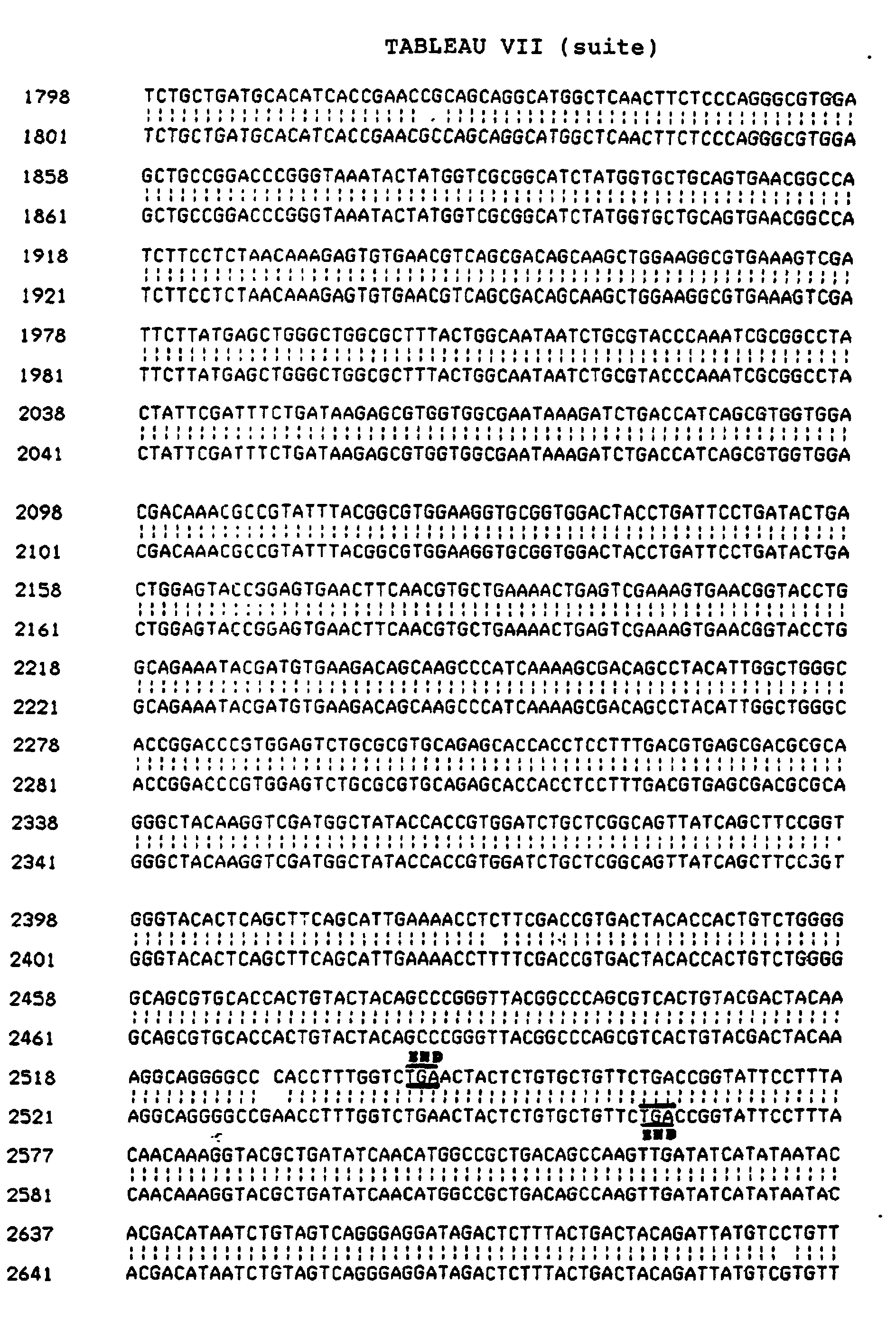

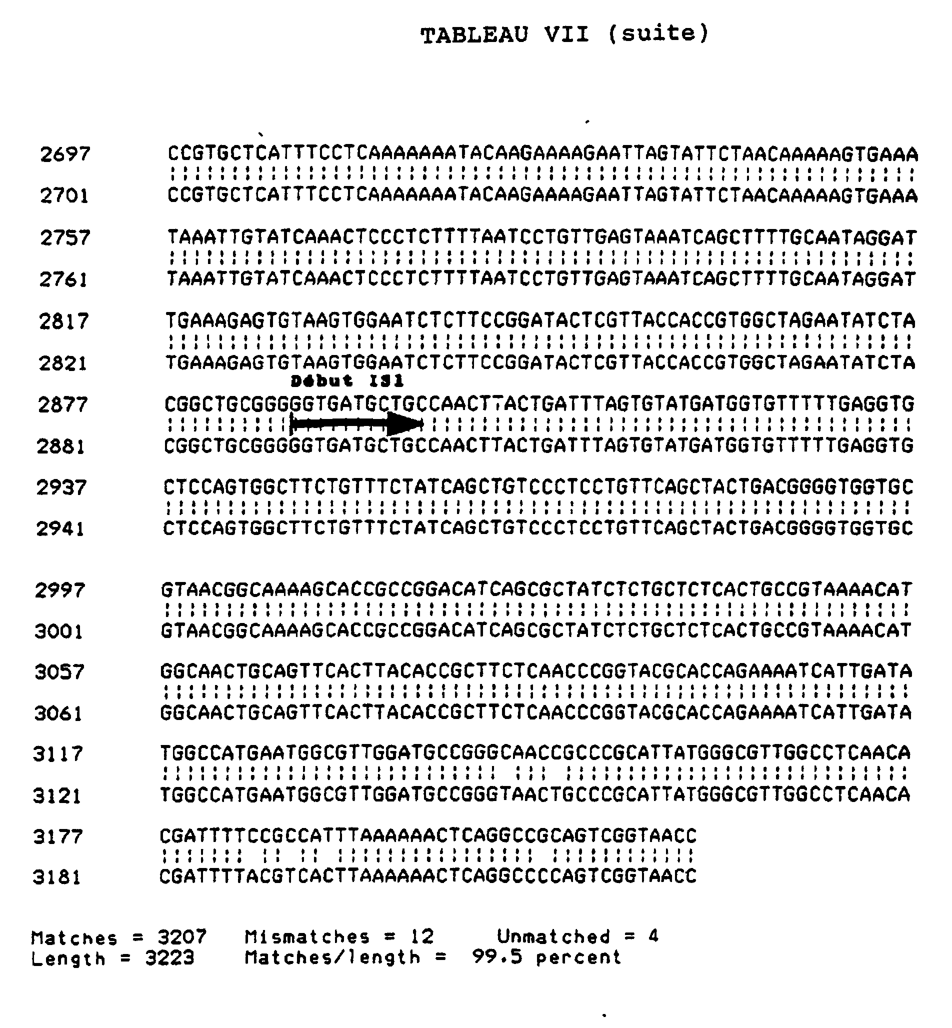

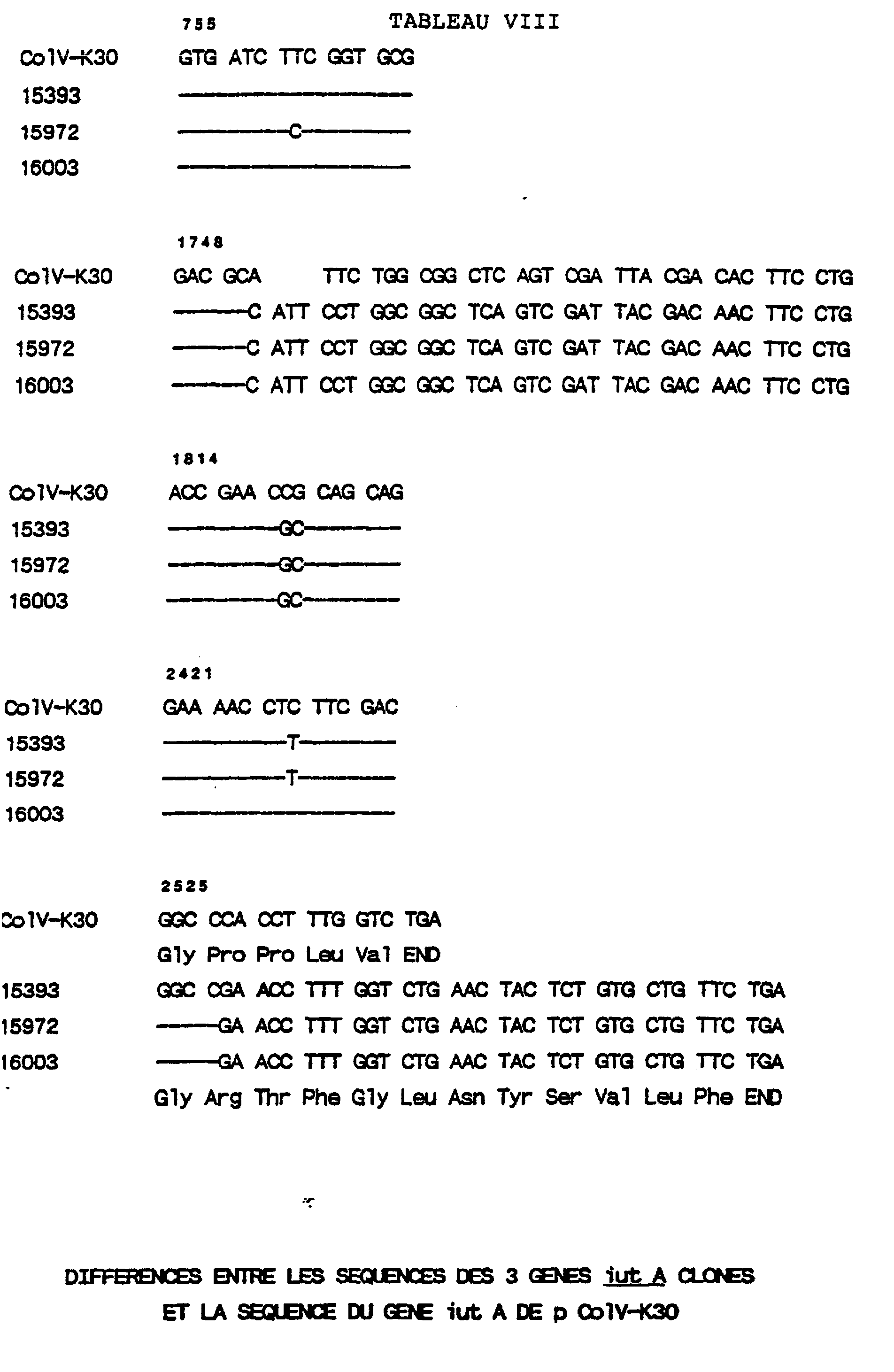

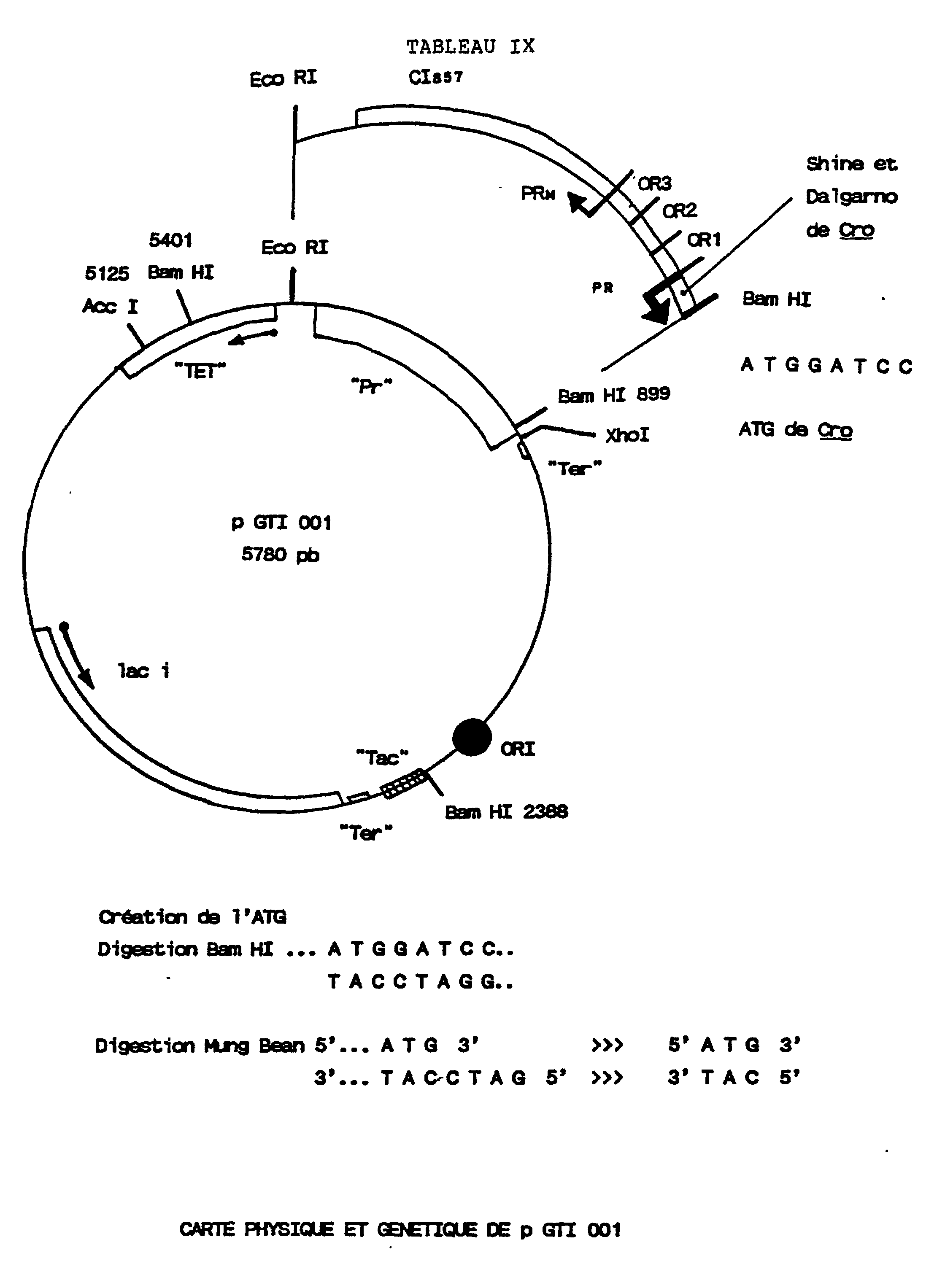

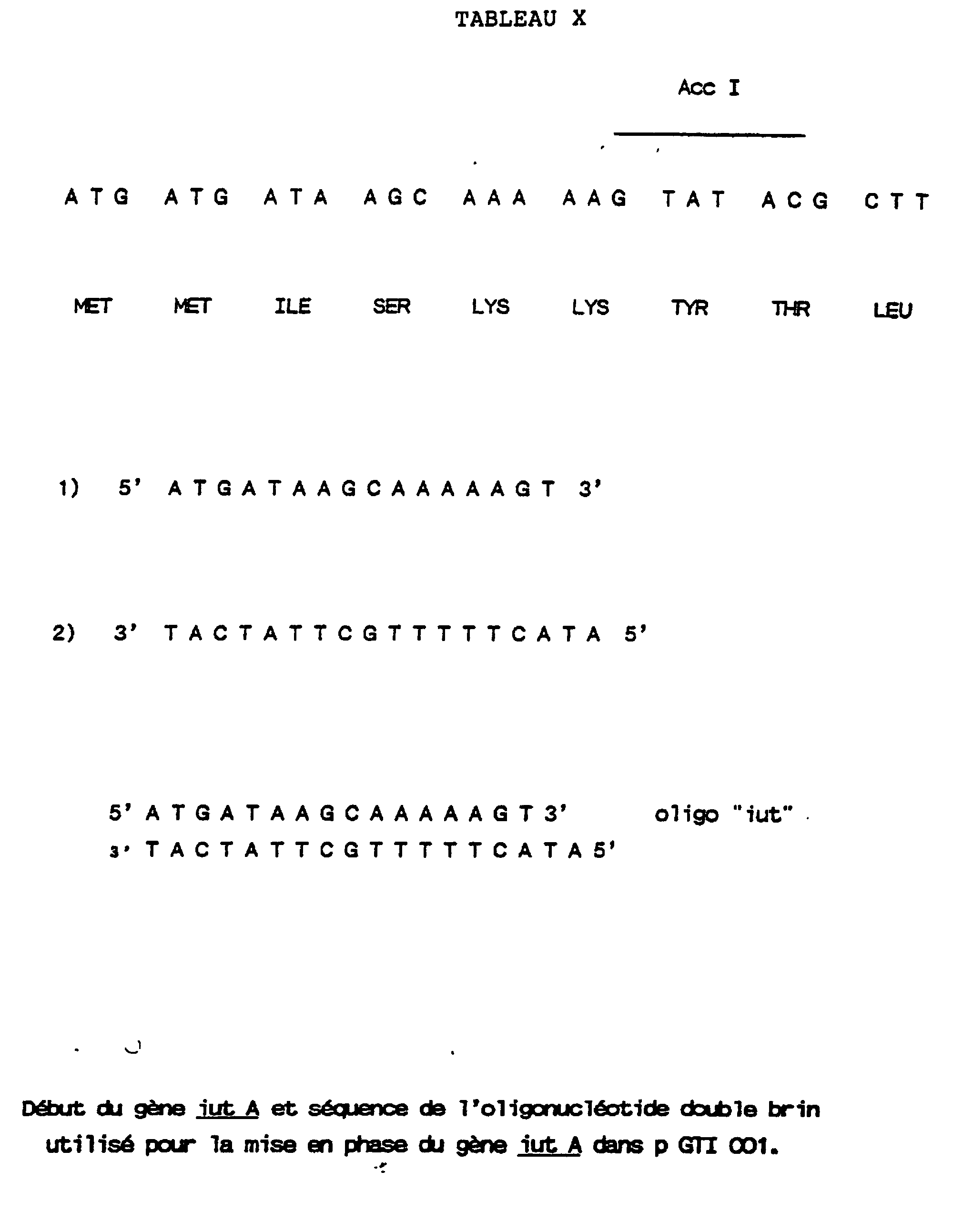

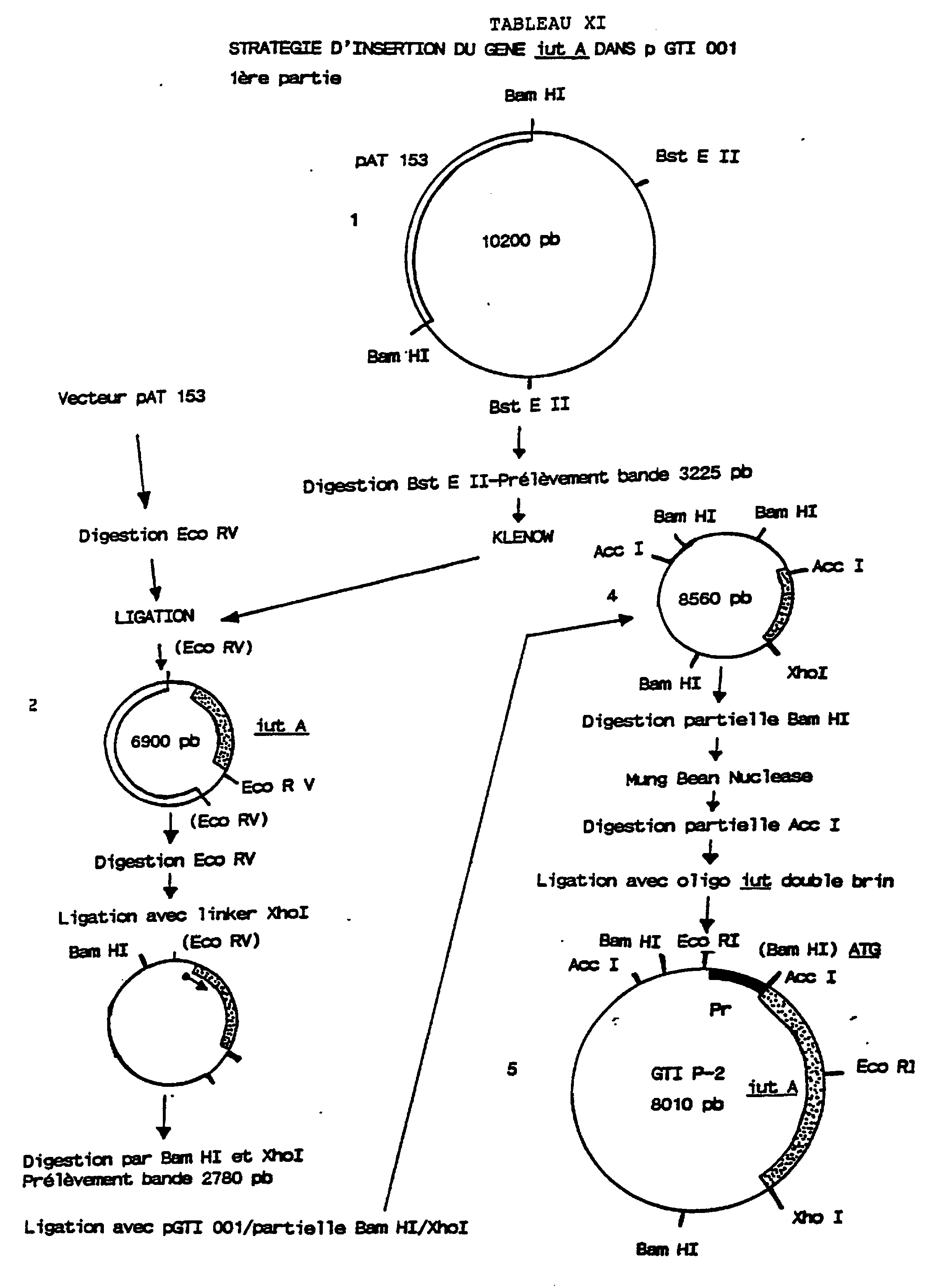

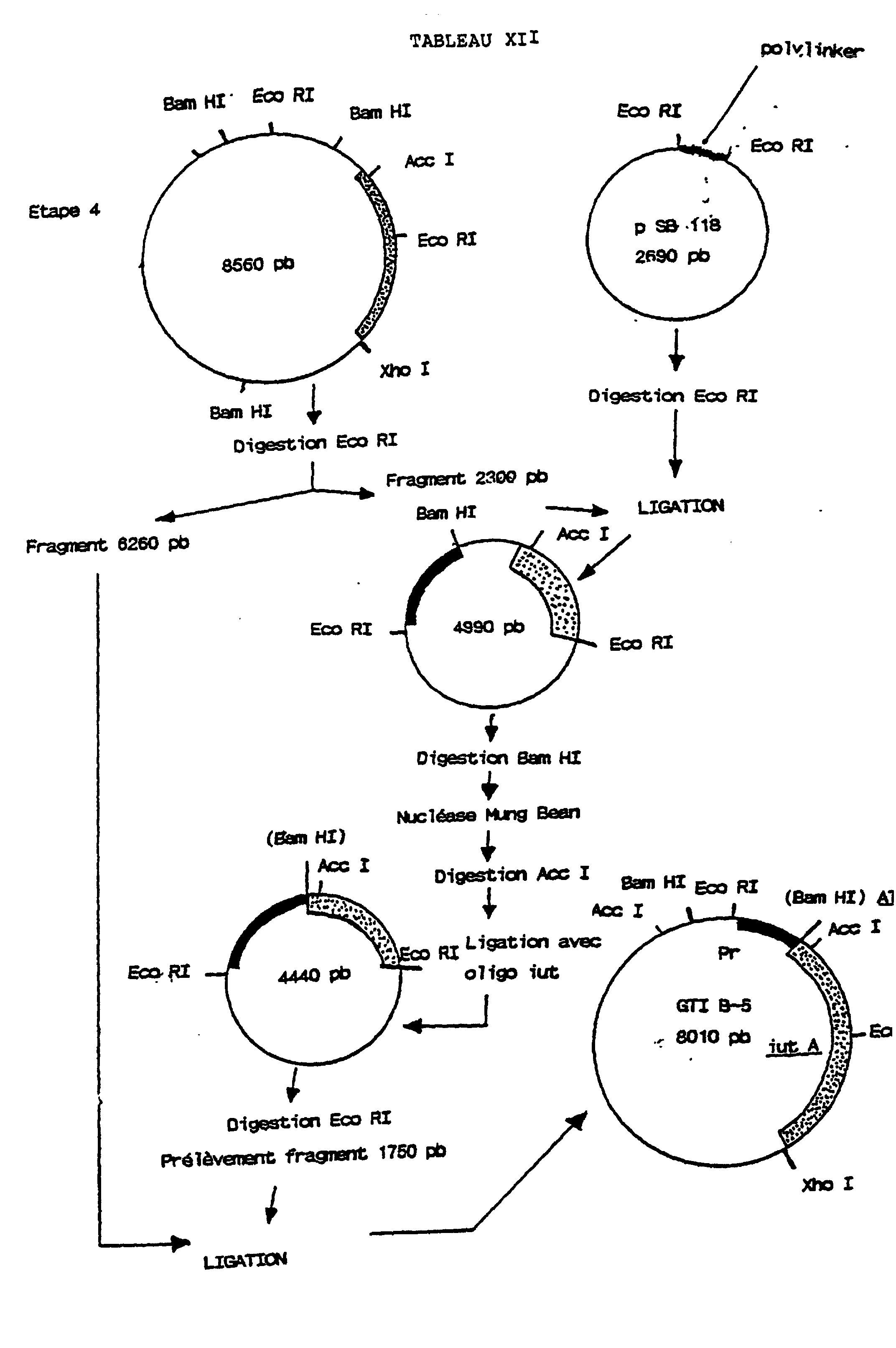

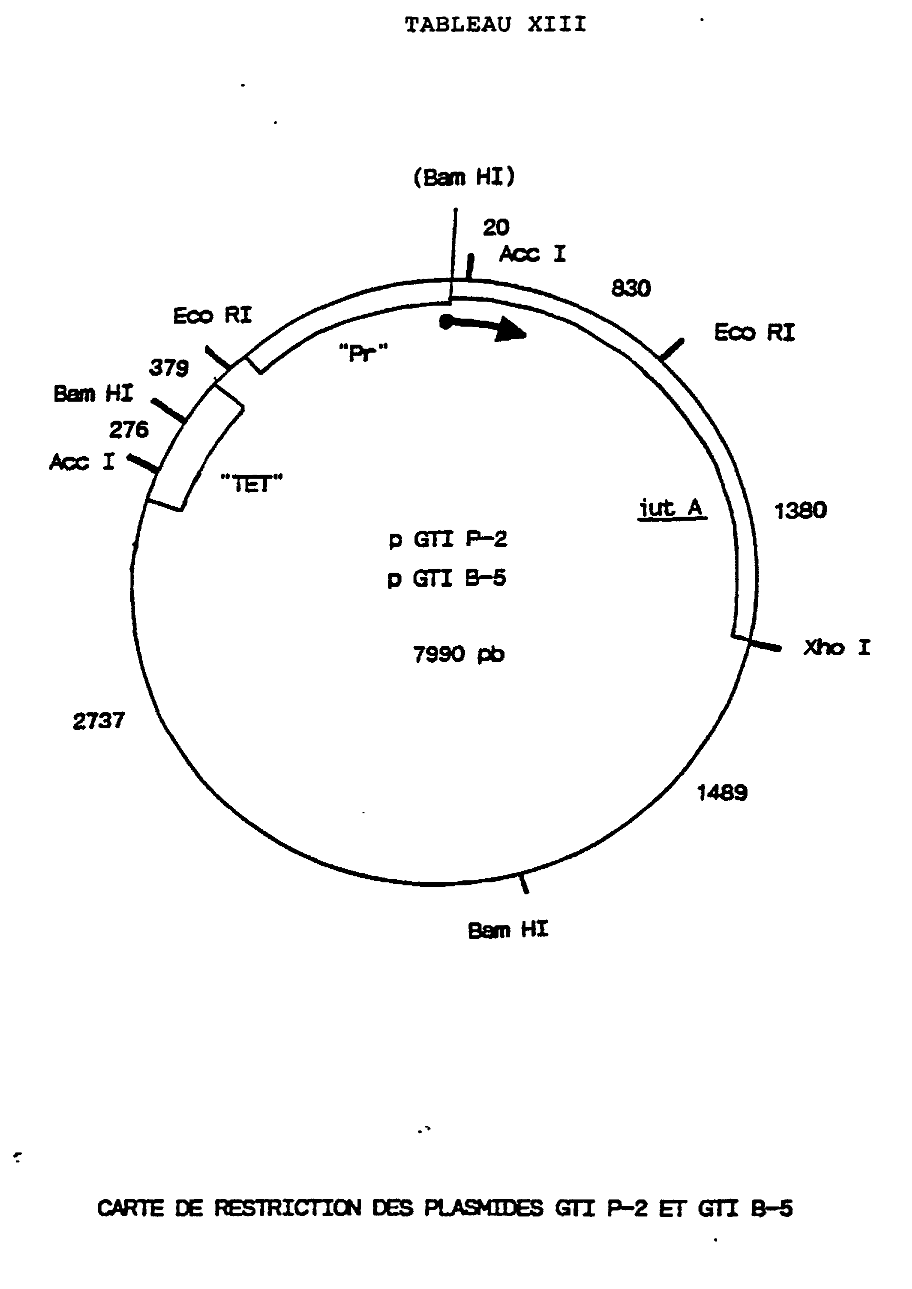

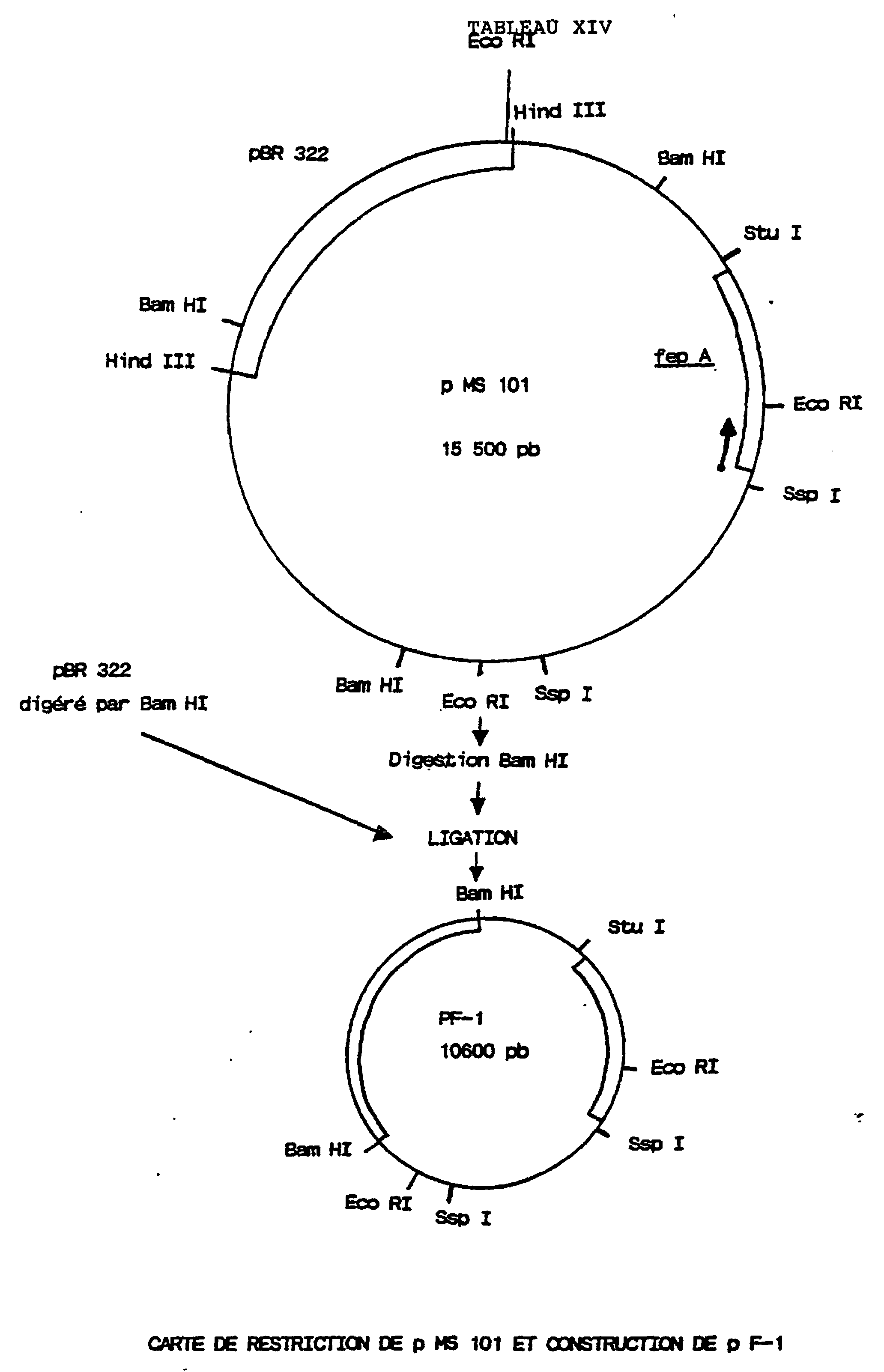

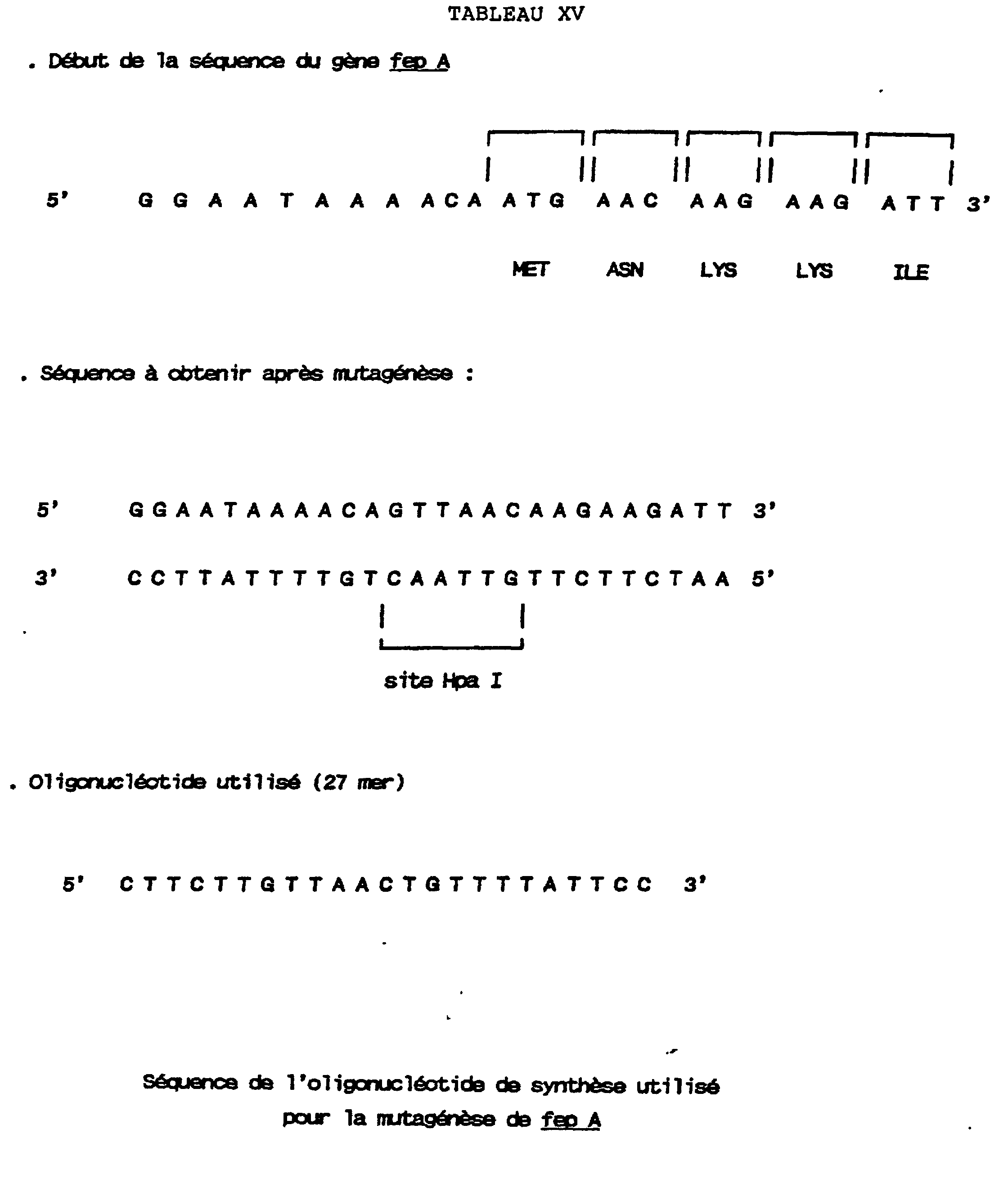

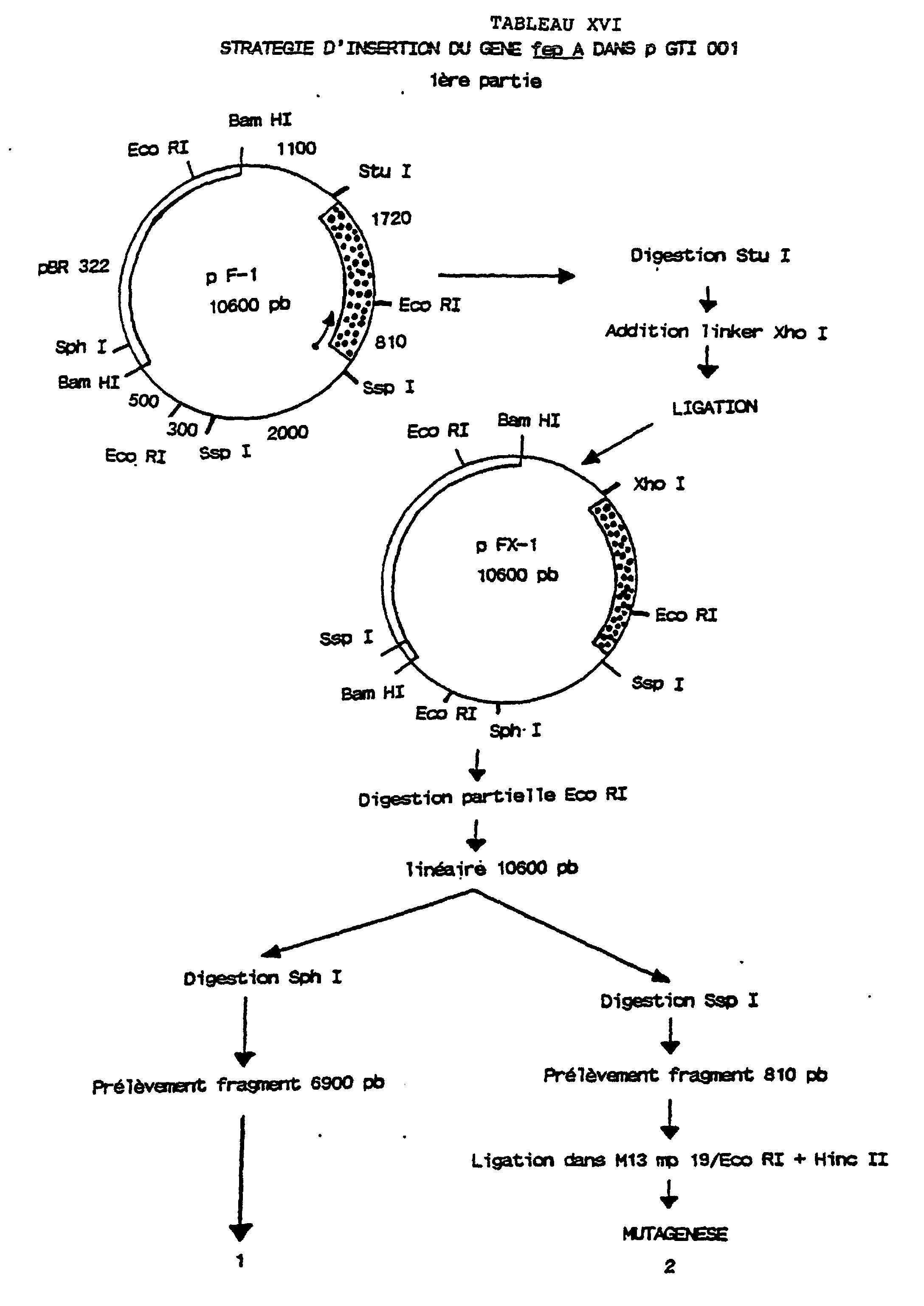

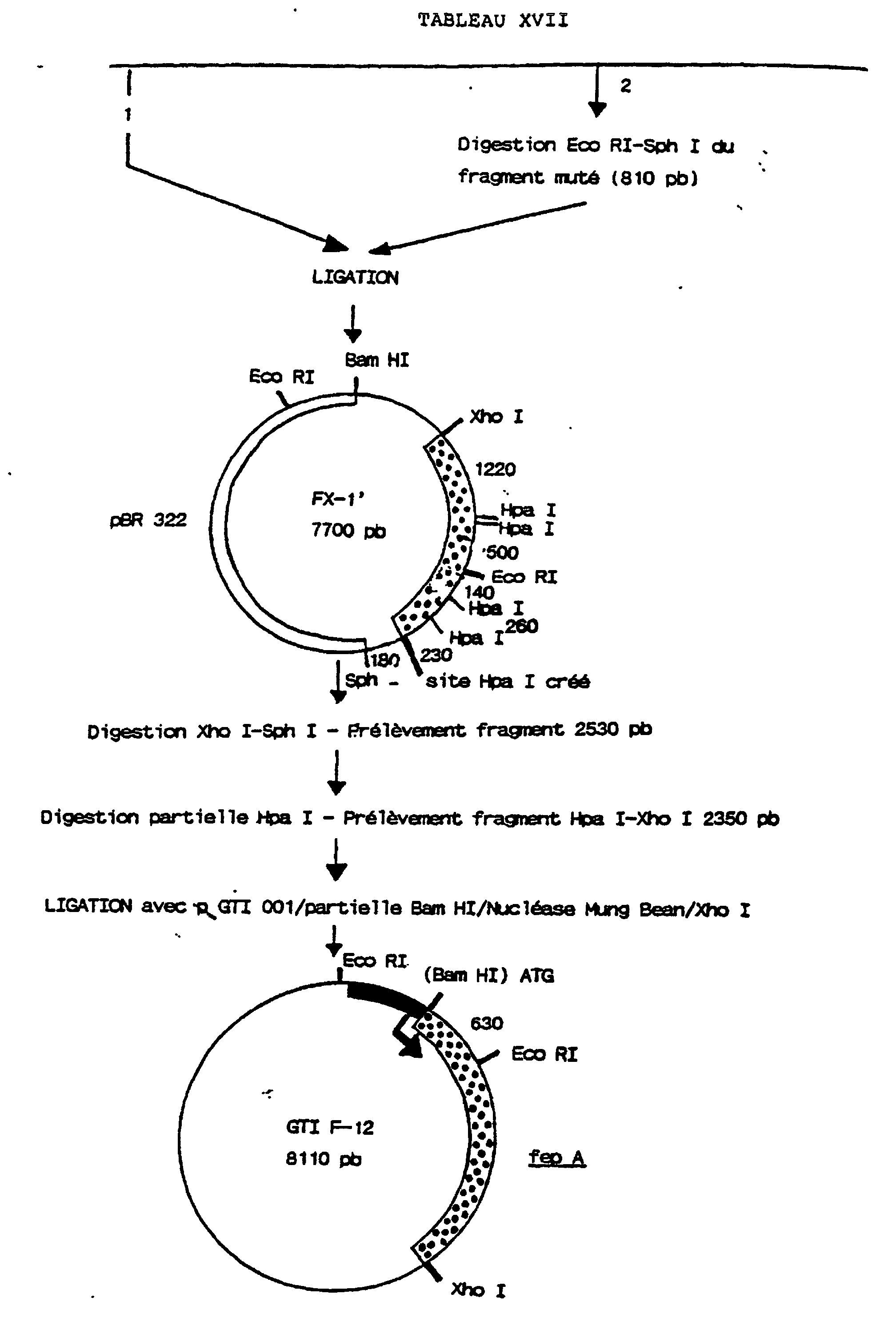

### BIBLIOGRAPHIE

(1) BINDEREIF A., BRAUN V. et HANTKE, J. Bacteriol. 150, 1472-1475 (1982)
(2) BINDEREIF A et NEILANDS I.B., J. Bacteriol 153, 1111-1113 (1983)
(3) BIRNBOIM H.C. et DOLY J., Nucleic Acids Res. 7, 1513-1523 (1979)
(4) BOLIN C.A. et JENSEN A.E., Infect. Imm. 55, 1239-1242 (1987)
(5) BYERS B.R., p. 111-116, "Iron transport in microbes, plants and animals", G. WINKELMAN, D. VAN DER HELM et J.B. NEILANDS, VCH Publishers Weinheim, FRG.
(6) CODERRE P. et EARHARDT C.F., "Characterization of a plasmid carrying the Escherichia coli K12, ent D, fep A, fes et ent F" gènes FEMS Microbiol. Letters 25, 111-116 (1984).
(7) COULTON J.W., Biochim. Biophys. Acta 717, 154, 162 (1982)
(8) DE GRAAF F.K., TIEZE G.A., WENDELAAR BONGA S. et STOUTHAMER A.H., J. Bacteriol. 95, 631-640 (1968)
(9) DE GRAAF F.K., GOEDVOLK-DE GROOT et STOUTHAMER A.H., Biochim. Biophys. Acta, 221, 566-575 (1970)
(10) ENGWALL E. et PERLMANN, J. Immunol. 109, 129-135 (1972)
(11) FLEMING T.P., NAHLIK M.S., NEILANDS J.B. et Mc INTOSH M.A., Gene, 34, 47-54 (1985)
(12) FISS E.H., STANLEY-SAMUELSON P. et NEILANDS J.B., Biochemistry, 21, 4517-4522 (1982)
(13) GRIFFITHS E., "Iron and infection : molecular, physiological and chemical aspects", J.J. BULLEN et E. GRIF-FITH, JOHN WILEY & SONS LTD, CHICHESTER ENGLAND.
(14) HERRERO M., DE LORENZO V. et NEILANDS J.B., J. Bacteriol 170, 56-64 (1988).
(15) HOLLIFIELD W.E. et NEILANDS, Biochemistry 17, 1922-1928 (1978) .
(16) KADO C.I. et LIU S.T., J. Bacteriol. 145, 1365-1373 (1978)
(17) KRONE W.J.A., OUDEGA B., STEGEHUIS F. et DE GRAAF F.K., J. Bacteriol 153, 716-721 (1983).
(18) KRONE W.J.A., STEGEHUIS F., KONINGSTEIN G., VAN DOORN C., ROSENDAL B., DE GRAAF F.K. et OUDEGA B., FEMS Microbiol. 26, 153 161 (1985)
(19) LAIRD A.J. et YOUNG I.G., Gene 11, 359-366 (1950)
(20) LOWRY O.H., ROSEBROUGH N.J., FARRA L.A. et RANDALL R.J., J. Biol. Chem. 193, 265-275 (1951)
(21) LUGTENBERG B., MEIJERS J., PETERS R., VAN DER HOEK P. et VAN ALPHEN L., FEBS Lett. 58, 254-258 (1975).
(22) LUNDIGRAN M.D. et KADNER R.J., J. Biol. Chem. 261, 10797-10801 (1986)
(23) MANIATIS T., FRITSCH E.F. et SAMBROOK J. (1982), Molecular cloning : on laboratory manual. Cold Spring Harbor Laboratory, Cold Spring Harbor NY.
(24) NAKAMAYE K. et ECKSTEIN, Nucl. Acids Res. 14, 9679-9698 (1986)
(25) OHTSUBO H. et OHTSUBO E., Proc. Natl. Acad. Sci, USA 75, 615-619 (1978)
(26) ROGERS H.J., SYNGE C et WOODS V.E., Antibacterial effect of scandium and indium complexes of enterochelin on Klebsiella pneumoniae. Antimicrob. Agents and Chemotherapy 18, 63-68 (1986)
(27) ROGERNS H.J., "Iron transport in microbes, plants and animais" G. WINKELMAN, D. VAN DER HELM, et J.B. NEILANDS, p. 223-233 (1987)-VCH Publishers, Weinheim, FRG
(28) SANGER F., NICKLEN S. et COULSON A.R., Proc. Natl. Acad. Sci. USA 74, 5463-5467 (1977).
(29) SCHNAITMAN C.A., J. Bacteriol 108, 545-552 (1971)
(30) SIMON E.H. et TESSMAN I., Proc. Natl. Acad. Sci. USA 50, 526-532 (1963)
(31) SOUTHERN E., J. Mol. Biol. 98, 503-517 (1975)
(32) TAYLOR J.W., SCHMIDT W., CROSSTICK R., OKRUSZEK A. et ECKSTEIN F., Nucl. Acids Res. 13, 8749-8764 (1985)
(33) TAYLOR J.W., OTT J. et ECKSTEIN F, Nucl. Acids Res. 13, 8765-8785 (1985)
(34) TOWBIN H., STAEHELIN T. et CORDON J., Proc. Natl. Acad. Sci USA 76; 4350-4354 (1979)
(35) VAN TIEL-MENKVELD G.J., OUDEGA B., KEMPERS O. et DE GRAAF F.K., FEMS, Microbiol. Lett. 12, 373-380 (1981)
(36) VAN TIEL-MENKVELD G.J., VELTKAMPF E. et DE GRAAF F.K., J. Bacteriol. 146, 41-48 (1981)
(37) WILLIAMS P.H., infect. Immun. 26, 925-932 (1979)
(38) YANISCH-PERRON C, VIEIRA J. et MESSING J., Gene 33, 103-119 (1985)

## Revendications

1. Utilisation de (a) bactéries entières obtenues par culture dans un milieu dans lequel on réduit suffisamment la teneur en fer, ce qui permet. d'obtenir une expression accrue de protéines de la membrane externe régulées par le fer et dont certaines sont des récepteurs de sidérophores ou de transferrines, ou (b) des fragments de ces bactéries, lesdits fragments intégrant des protéines récepteurs de transferrines ou sidérophores, telles que notamment la protéine Iut A et/ou Fep A et/ou leurs précurseurs proIut A et/ou proFep A, ou (c) des protéines récepteurs de transferrines ou sidérophores, et notamment les protéines Iut A et Fep A, extraites de ces bactéries, pour la fabrication, à titre de principe actif, d'une composition thérapeutique permettant une immunisation contre des bactéries septicémiques,

2. Utilisation selon la revendication 1, **caractérisé en ce que** les bactéries appartiennent au groupe formé par les entérobactéries des familles Escherichia coli, Klebsiella, Salmonella thyphimurium, Shigella.

3. Utilisation selon la revendication 2, **caractérisé en ce que** les sidérophores excrétés par les bactéries, sont des sidérophores aérobactine et/ou entérobactine.

4. Utilisation selon la revendication 3, **caractérisé en ce que** les protéines exprimées sont les protéines Iut A et/ou Fep A.

5. Utilisation selon l'une quelconque des revendications précédentes, dans laquelle lesdites bactéries entières sont des bactéries obtenues par culture dans un milieu dans lequel on réduit 1a teneur en fer à l'aide d'une protéine forte chélatrice du fer, telle qu'une lactoferrine, ladite protéine forte chélatrice du fer étant présente à une concentration d'au moins 250 µg/ml.

## Patentansprüche

1. Verwendung von (a) ganzen Bakterien, die durch Kultur in einem Medium erhalten werden, in dem der Gehalt an Eisen genügend verringert wird, dass man eine erhöhte Expression von Außenmembranproteinen erhalten kann, die durch Eisen reguliert werden und von denen bestimmte Rezeptoren für Siderophore oder Transferrine sind, oder (b) Fragmenten dieser Bakterien, wobei diese Fragmente Rezeptorproteine für Transferrine oder Siderophore beinhalten, wie insbesondere das Protein IutA und/oder FepA und/oder deren Vorläufer proIutA und/oder proFepA, oder (c) Rezeptorproteinen für Transferrine oder Siderophore und insbesondere die Proteine IutA und/oder FepA, die aus Bakterien extrahiert sind, als Wirkstoff zur Herstellung einer therapeutischen Zusammensetzung, die eine Immunisierung gegen Septikämiebakterien ermöglicht.

2. Verwendung nach Anspruch 1, **dadurch gekennzeichnet, dass** die Bakterien aus der Gruppe stammen, die von den Enterobakterien der Familien Escherichia coli, Klebsiella, Salmonella typhimurium, Shigella gebildet wird.

3. Verwendung nach Anspruch 2, **dadurch gekennzeichnet, dass** die durch die Bakterien ausgeschiedenen Siderophore Aerobactin- und/oder Enterobactin-Siderophore sind.

4. Verwendung nach'Anspruch 3, **dadurch gekennzeichnet, dass** die exprimierten Proteine die Proteine IutA und/oder FepA sind.

5. Verwendung nach einem der vorhergehenden Ansprüche, wobei die ganzen Bakterien Bakterien sind, die durch Kultur in einem Medium erhalten werden, in dem der Gehalt an Eisen mit einem Eisen stark chelatisierenden Protein, wie einem Lactoferrin, verringert wird, wobei das Eisen stark chelatisierende Protein in einer Konzentration von mindestens 250 µg/ml vorliegt.

## Claims

1. Use of (a) whole bacteria obtained by cultivation in a medium in which the iron content has been sufficiently reduced, making it possible to achieve increased expression of proteins of the outer membrane which are regulated by iron and some of which are receptors for siderophores or transferrins, or (b) fragments of these bacteria, said fragments incorporating proteins which are receptors for transferrins or siderophores, such as in particular the protein Iut A and/or Fep A and/or their precursors proIut A and/or proFep A, or (c) proteins which are receptors for transferrins or siderophores, and notably the proteins Iut A and Fep A, extracted from these bacteria, for the production, as active ingredient, of a therapeutic composition which can be used for immunisation against septicaemic bacteria.

2. Use according to claim 1, **characterised in that** the bacteria belong to the group formed by the enterobacteria of the families *Escherichia coli, Klebsiella, Salmonella thyphimurium, Shigella.*

3. Use according to claim 2, **characterised in that** the siderophores excreted by the bacteria are the siderophores aerobactin and/or enterobactin.

4. Use according to claim 3, **characterised in that** the proteins expressed are the proteins Iut A and/or Fep A.

5. Use according to any one of the preceding claims, wherein said whole bacteria are bacteria obtained by cultivation in a medium in which the iron content has been reduced by means of a strong iron-chelating protein such as a lactoferrin, said strong iron-chelating protein being present in a concentration of at least 250 µg/ml.
